(19) **Europäisches Patentamt**
**European Patent Office**
**Office européen des brevets**

(11) **EP 4 398 053 A1**

(12) **EUROPEAN PATENT APPLICATION**

(43) Date of publication:
**10.07.2024 Bulletin 2024/28**

(21) Application number: **24150601.3**

(22) Date of filing: **05.01.2024**

(51) International Patent Classification (IPC):
***G05B 13/04*** (2006.01)     ***G05B 17/00*** (2006.01)
***G06F 17/11*** (2006.01)

(52) Cooperative Patent Classification (CPC):
**G05B 13/04; G05B 17/00;** G05B 2219/41124;
G05B 2219/41133; G06F 17/11; G16H 50/50

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **05.01.2023 PT 2023118456**

(71) Applicant: **Universidade de Aveiro**
**3810-193 Aveiro (PT)**

(72) Inventors:
• **Soares dos Santos, Marco Paulo**
**3810-193 Aveiro (PT)**
• **Cabral Bernardo, Rodrigo Miguel**
**3810-193 Aveiro (PT)**
• **Marado Torres, Delfim Fernando**
**3810-193 Aveiro (PT)**
• **Ruivo Herdeiro, Carlos Alberto**
**3810-193 Aveiro (PT)**

(74) Representative: **Patentree**
**Edificio Net**
**Rua de Salazares, 842**
**4149-002 Porto (PT)**

(54) **CONTROL METHOD FOR TRAJECTORY TRACKING OF A DYNAMIC SYSTEM AND RESPECTIVE DEVICE**

(57)    The present document discloses a control method for trajectory tracking of a dynamic system (2) having an input $u(t)$ and having an output $y(t)$, comprising the steps: obtaining a signal error $e(t)$ from the difference between a reference signal $u(t)$ and the output signal $y(t)$; obtaining, by a controller (1,4), a control value $c_{BN}(t)$ determined by a black hole (BH) gravitational potential field of a black hole with mass $M(t)$, wherein the reference signal $u(t)$ is the black hole centre, wherein the signal error $e(t)$ is a distance of a current position $y(t)$ to the black hole centre $u(t)$; wherein the output signal $y(t)$ is a current position within a black hole Schwarzschild radius of the black hole, and where the black hole mass $M(t)$ includes a factor of matter accretion; and inputting the control value $c_{BN}(t)$ into the dynamic system (2). It is further disclosed a respective controller and dynamic system (2) comprising a controller for trajectory tracking.

$$M(t)\frac{(a\,e)^{p-1}}{\left((a\,e)^q+r_0^q\right)^{\frac{p}{q}}}$$

Fig. 2

EP 4 398 053 A1

**Description**

**TECHNICAL FIELD**

The present disclosure relates to an automatic control method and respective controller device. The control laws are formulated using mathematical models of physical dynamics found in Black Holes, such that desired control requirements can be achieved. The controller is incorporated into a closed-loop structure with negative feedback, and (at least) the dynamic system under control and a sensing system.

**BACKGROUND**

Up to date, control methods do not consider the dynamics occurring in natural phenomena (non-inspired control) or only consider some dynamics occurring in biological structures (bioinspired control). Many non-inspired control methods were already proposed, such as the Proportional, Integral and Derivative (PID) control, predictive control, optimal control, and sliding mode control. However, these are "artificial" methods do not consider the natural environment where they are operating. Therefore, they do not have the ability to perform a full integration that minimizes the amount of energy required to ensure specific trajectories. The best-known bioinspired control methods are Artificial Neural Networks (ANN) and Fuzzy Logic (FLC). The relevant limitations of ANN include: (i) lack of transparency, interpretability, generalization and effectiveness; (ii) it is hard to define the minimal amount of data required to train the networks, such that desired control performances can be ensured; (iii) high processing times and high hardware requirements. Concerning the limitations of FLC, the following ones must be highlighted: (a) lack of interpretability; (b) it is hard to define the shape of membership functions; (c) the fuzzy rule base introduces intuition and redundancy in objective control problems; (d) high processing times.

Black Hole (BH) dynamics have been used in model identification, optimization of parameters from the dynamic systems under control (2), and tuning of parameters from PID controllers, FLC and other non-linear controllers.

Besides, human-related rules are the most fundamental basis of FLC, which can introduce intuition and redundancy in objective control problems, and require high computational processing ability. This limitation is commonly solved using optimisation algorithms to find which parameters guarantee the best performances, but such algorithms are also human-related.

On the other side, cancer chemotherapy is a worldwide well-established treatment for a wide range of cancers, even though its effectiveness as its effectiveness is highly dependent on tumour-cell, tissue microenvironment and organism-level heterogeneities [1-5]. Nevertheless, the problem is multifaceted: (i) such heterogeneity remains largely unrevealed, causing in many cases poor therapeutic outcomes. Included are the great amount of analytical models aiming to predict the tumour volume dynamics and to provide a good fit to tumour volume dynamics obtained from clinical measurements, as well as the significant variations of their parameters; (ii) standard empirical chemotherapy remains the current clinical methodology; (iii) discrete/intermittent dose administration (weekly basis, etc.) is still the preferential clinical therapeutic approach. As a result, poorly defined therapies are common, medication errors and dosage adjustments occur very often (recommendations can be scarce, mainly if other comorbidities exist), and the survival probabilistic paradigm imposes. This paradigm does not allow a clinical practice supported by a rational basis, even if targeted therapies (e.g. by delivering targeted anticancer agents), and some degree of personalized treatment (e. g. by adapting therapy based on each patient genetic profile) are used.

Classical control methods have also been used to optimize the drug delivery, but they have not achieved a confidence level to be translated to clinical practice, mainly due to their quite dependent effectiveness on accuracy of analytical models, or their serious limitations concerning estimation of relevant physiological states (mainly the tumour volume) throughout treatment (the current paradigm does not allow changing the model parameters frequently, e.g., on a daily basis), or their parametric uncertainties were analyzed in very narrow ranges (which have not considered the real large parametric changes in human patients), or their inherent non-negligible degree of intuition, or reduced personalized controllability due to discrete dose administration (even when a frequent administration schedule established) and to adaptive approaches based on limited model clustering. These are non-nature-inspired and "artificial" control approaches that neglect the rationality and effectiveness expressed in nature, consequently discrediting computationally-driven decision-making tool towards an uncertain future.

Furthermore, for this case of cancer therapy, the known approach establishes a probabilistic paradigm in which patients do not know if they will survive, but only the survival probability (e.g. 5-year survival rate of $\approx$10% in pancreatic cancer [6]).

The document CN110880031A discloses a feature selection method for a grey wolf optimization algorithm, incorporating a fused random black hole strategy, comprising initializing a grey wolf population, updating wolf positions using the random black hole strategy, adjusting positions based on the relative distance between key wolves, and predicting optimal solutions. This method is applied to the intrusion detection process. However, this document refers to an optimization method, which is a searching method, and, therefore, it is totally different from a control method, which is established to change the states of dynamic systems according to desired trajectories.

These facts are disclosed in order to illustrate the technical problem addressed by the present disclosure.

**GENERAL DESCRIPTION**

This invention concerns a control method that uses physical dynamics occurring in Black Holes in non-linear controllers. It is not based in natural phenomena occurring in biological structures. The controller (1) is implemented through analogies between the control system and the physical system characterizing the Black Hole. The response $c_{BH}$ of the controller is obtained by applying a control law inspired in one or more physical phenomena related and/or associated with Universal Gravitation (one of the four fundamental interactions in nature). The controller (1) must be incorporated into a closed-loop structure with negative feedback, which must also include the system under control (2) and a sensing system (3). The reference $u$ is associated to the error e, obtained by the difference between the reference $u$ and the output $y$ of the system under control (2). This error is minimized by the controller (1) operation, through de application of a control signal $c_{BH}$ to the system under control (2). The controller (1) parameters must be optimized according to the type of system under control (2) and the performance requirements (maximum trajectory tracking error, maximum stationary error, maximum rise time, maximum settling time, etc.). This control method can be used either for tracking trajectories between different system states of the system under control (2), or to keep certain system state(s) unchanged.

A nonlinear controller is disclosed and analysed for trajectory tracking problems related to generic second-order linear systems. An equilibrium point was determined, as well as the necessary conditions to guarantee the system's stability. Numerical examples highlight the ability of this BH controller to provide significant performance improvements over the PID family controllers, even if the parameter optimisation is carried out using the heuristic BH search algorithm. Promising results are herein reported, highlighting the potential of BH-inspired nature-based control for highly sophisticated controllers for multiples control problems.

This is a control method that can be used in all technologies requiring trajectory tracking, as well as in technologies related to the design and simulation of dynamic systems. Among these technologies, one must highlight those related to position and speed control of electric, hydraulic and pneumatic servomechanisms, temperature in chemical and thermofluid systems, delivery systems of drugs in biomedical systems, e.g. to reduce tumour volumes, etc.

This control method solves three problems: (i) it overcomes the intuition and lack of interpretability found in bioinspired control methods; (ii) it allows the integration into natural environment where there are masses/weight; and (iii) it can reduce the computational costs when compared to bioinspired control methods.

Black holes are trapped regions in spacetime. This means that (classically), neither matter nor light can escape from a black hole. The (virtual) boundary of the trapped region is called the event horizon. Crossing the horizon, space and time interchange. This is why black holes cannot be escaped from, once the horizon is crossed. Inside a black hole, the future is inwards.

Mathematically, black holes emerged as solutions of the General Theory of Relativity postulated by Albert Einstein in 1915. There are two influential black hole solutions thereof. The first one, published by Karl Schwarzschild in 1916, describes a perfectly spherical, non-spinning black hole. The second one - actually a generalisation of the first - was found by Roy Kerr in 1963, and describes a spinning black hole. Subsequently, a series of mathematical theorems - the black hole uniqueness theorems - showed that the Kerr solution is the most general, physical, black hole solution in the absence of any matter sources. This result became the pillar of the Kerr hypothesis that all black holes in the Universe are well described by the Kerr solution.

In Astrophysics, these mathematical solutions describing black holes were first called upon to explain bright radio and X-ray sources in the sky, which required extraordinary energy sources. In the last decade, with the detection of gravitational waves, black hole imaging of M87* and Sgr A*, and the tracing of orbits around Sgr A* (the supermassive black hole at the centre of the Milky Way), a consensus emerged in the scientific community that black holes are abundant, key players in the Cosmic scene.

At the current precision of observations, there is yet no clear tension between observations and the Kerr hypothesis. However, black holes contain the seeds of their own demise. Inside the Kerr solution there is spacetime singularity, wherein General Relativity ceases to be predictive. Even more dramatically, Roger Penrose showed that such singularities are inevitable inside black holes, for physically reasonable matter-energy.

To cope with this undesirable property, throughout the years models of black holes without a central singularity have been proposed, either as ad hoc models, or as solutions of the field equations with exotic matter sources. Such models are known as regular black holes.

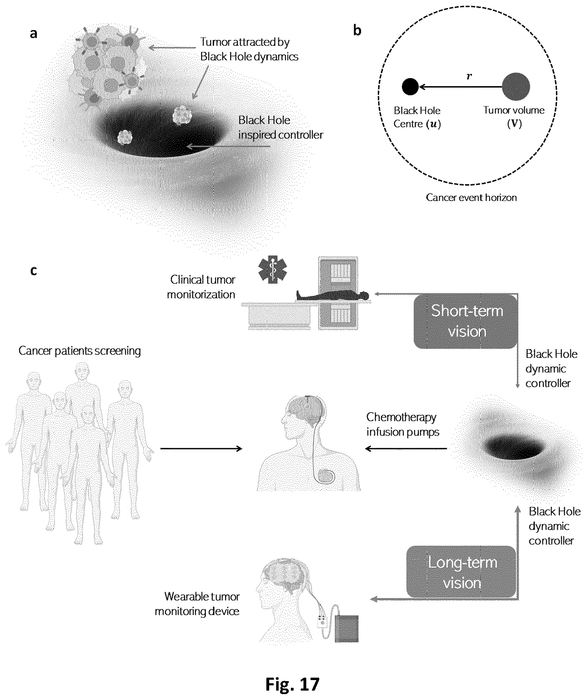

Furthermore, this solution provides a BH inspired dynamics for cancer therapies, by considering that tumour masses are trapped and/or destroyed, regardless the idiosyncrasies of patients (Fig. 17a). Once tumours are found inside the cancer event horizon (the cancer-adapted Schwarzschild radius), treatment based on BH dynamics will ensure the patient survival whatever the tumour volume and related dynamics, before reaching a metastatic state. The BH-cancer bridge is here defined according to a realistic analogy between control principles and BH phenomena (Fig. 17b):

desired tumour volume trajectory ($u$) $\mapsto$ BH centre ( $u \in \mathbb{R}^+$ ); drug administration ($T$) $\mapsto$ gravitation ( $T \in \mathbb{R}^+$ ); real tumour volume ($V$) $\mapsto$ position of the mass attracted to BH ( $V \in \mathbb{R}^+$ ); volume error ($r$) $\mapsto$ distance between the mass and the BH centre ($r \in \mathbb{R}^+$ ).

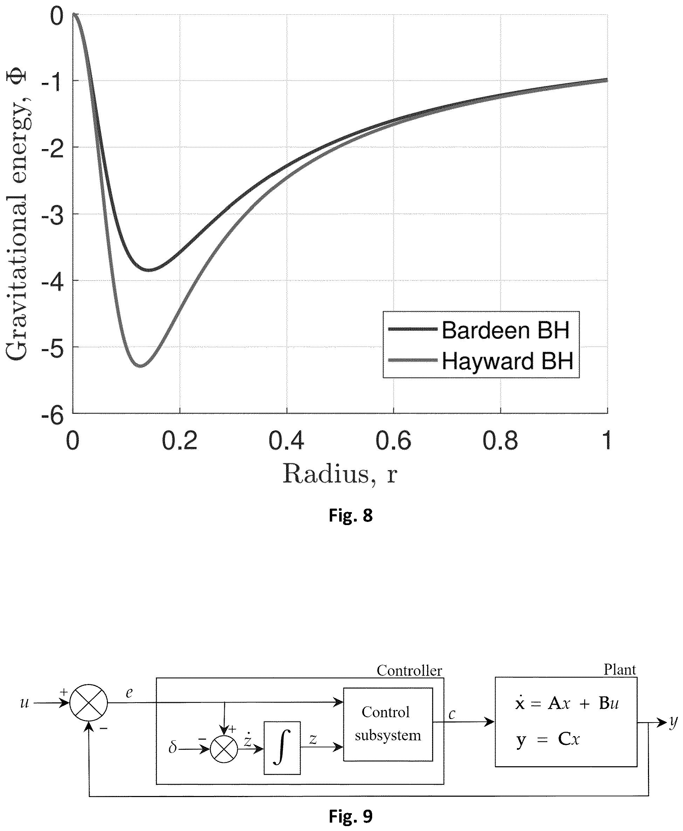

The present disclosure can be applied according to both a short-term vision and a long-term vision (Fig. 17c). Firstly, both visions require a rigorous screening to identify which patients can undergo this new treatment paradigm, namely if there is no urgency to perform the tumour removal and/or to control other comorbidities. The short-term vision can be applied using current clinical imaging technology (e.g. magnetic resonance imaging, MRI, computed tomography scan, CT, and single-photon emission computed tomography, SPECT) to monitor the tumour growth dynamic profile for a short duration, such that the analytical models and their parameters can be identified to optimize the BH controller parameters. The drug administration must be carried out in a continuous basis, using drug releasing devices for either extracorporeally (e.g. for breast and brain cancers) or intracorporeally (e.g. pancreatic cancer) controlled delivery. Models can be updated throughout treatment, such that the BH controller can accordingly change the drug dosing. The long-term vision only differs from the short-term vision regarding the use of (extracorporeally or intracorporeally) in situ sensing system with ability to continuously provide the tumour volume dynamics. This groundbreaking study found that once the CH controller is firstly optimized, according to the initial tumour volume and desired treatment trajectory (Fig. 8d), the BH controller can always reach a free-tumour state whatever the tumour growth dynamics exhibited by each patient, including for different chemotherapy drugs.

The present document discloses a control method for trajectory tracking of a dynamic system having an input $u(t)$ and having an output $y(t)$, comprising the steps: obtaining a signal error $e(t)$ from the difference between a reference signal $u(t)$ and the output signal $y(t)$; obtaining, by a controller, a control value $c_{BN}(t)$ determined by a black hole (BH) gravitational potential field of a black hole mass $M(t)$, where the reference signal $u(t)$ is the black hole centre, where the signal error $e(t)$ is a distance of a current position $y(t)$ to the black hole centre $u(t)$, where the output signal $y(t)$ is a current position within a black hole Schwarzschild radius of the black hole, and where the black hole mass $M(t)$ includes a factor of matter accretion; and inputting the control value $c_{BN}(t)$ into the dynamic system.

In an embodiment, the control method further comprises the steps: obtaining the signal error $e(t)$ from the difference between the reference signal $u(t)$ and the output signal $y(t)$; obtaining, by the controller, the control value $c_{BN}(t)$ determined by the equation:

$$c_{BN}(t) = M(t) \frac{\left(ae(t)\right)^{p-1}}{\left(\left(ae(t)\right)^q + r_0^q\right)^{p/q}}$$

wherein $M(t)$ corresponds to the factor of matter accretion, $p$, $q$ and $a$ are pre-defined parameters to define a convergence potential, and $r_0$ is a pre-defined constant to ensure non-singular solution or solutions, and wherein $p$ and $q$ are even and positive; and inputting the control value $c_{BN}(t)$ into the dynamic system.

In an embodiment, the factor of matter accretion $M(t)$ is determined by $M(t) = M_0 + k_1 exp(k_2(\int e\, dt)^2)$, wherein $M_0$ is an initial mass parameter of the BH controller, and $k_1$ and $k_2$ are predefined convergence parameters.

In an embodiment, the control method further comprises a hyperparameter tuning step, in particular a preparatory hyperparameter tuning step, in particular using a Nelder-Mead simplex and/or BH optimisation algorithm (an optimization method using the disclosed control method).

The BH optimisation algorithm, i.e., not a control method, it is inspired by the behaviour of stars around black holes. The search agents are represented by stars, and the one with the highest fitness value is established as the black hole. The black hole agent attracts the others, imitating the behaviour of a real black hole. A star is absorbed when it crosses the so-called Schwarzschild radius, which results in the removal of the agent from the search space. To maintain a balance in the number of agents, a new star is added at a random position of the search space. Throughout the iterations, if any star becomes fitter than the black hole, then the role of black hole will be performed by that star in the next iteration. After a predefined stopping criterion, the optimal solution is given by the agent that is the black hole in the last iteration.

In an embodiment, the dynamic system comprises a tumour growth dynamic model, wherein the input $u(t)$ is a tumour treatment parameter and the output $y(t)$ is a tumour growth metric, in particular a tumour volume.

It is also disclosed a controller for trajectory tracking of a dynamic system having an input $u(t)$ and having an output $y(t)$, comprising a data processor arranged to carry out the steps of: obtaining a signal error $e(t)$ from the difference between a reference signal $u(t)$ and the output signal $y(t)$; obtaining, by the controller, a control value $c_{BN}(t)$ determined by a black-hole gravitational potential field of a black hole with mass $M(t)$, where the reference signal $u(t)$ is the black hole centre, where the signal error $e(t)$ is a distance to the black hole centre $u(t)$, where the output signal $y(t)$ is a current position within a black hole Schwarzschild radius of the black hole, and where the black hole mass $M(t)$ includes a factor of matter accretion $M(t)$ and inputting the control value $c_{BN}(t)$ into the dynamic system.

In an embodiment, the data processor is further arranged to carry out the steps of: obtaining the signal error $e(t)$ from the difference between the reference signal $u(t)$ and the output signal $y(t)$; obtaining, by the controller, the control value $c_{BN}(t)$ determined by the equation:

$$c_{BN}(t) = M(t) \frac{\left(ae(t)\right)^{p-1}}{\left(\left(ae(t)\right)^{q} + r_0^{q}\right)^{p/q}}$$

wherein $M(t)$ corresponds to the factor of matter accretion, $p$, $q$ and $a$ are pre-defined parameters to define a convergence potential, and $r_0$ is a pre-defined constant to ensure non-singular solution or solutions, and wherein $p$ and $q$ are even; and inputting the control value $c_{BN}(t)$ into the dynamic system.

In an embodiment, the factor of matter accretion $M(t)$ is determined by $M(t) = M_0 + k_1 exp(k_2(\int e\, dt)^2)$, wherein $M_0$ is an initial mass parameter of the BH controller, and $k_1$ and $k_2$ are predefined convergence parameters.

It is further disclosed a dynamic system comprising a controller for trajectory tracking of the dynamic system (2) of any of the claims 5-7.

In an embodiment, the dynamic system is a linear system or a non-linear system linearized at particular operating points.

In an embodiment, the dynamic system is a non-linear system.

In an embodiment, the dynamic system comprises a tumour growth dynamic model, wherein the input u(t) is a tumour treatment parameter and the output y(t) is a tumour growth metric, e.g., a tumour volume.

## BRIEF DESCRIPTION OF THE DRAWINGS

The following figures provide preferred embodiments for illustrating the disclosure and should not be seen as limiting the scope of invention.

**Figures 1, 2, 3 and 4** schematically illustrate the block diagrams of the control system inspired by Black Hole dynamics.

**Figure 5** shows numerical results related to the response of the BH controller (4) and PID controller (6) to the 2nd order linear system when the sigmoid- logistic trajectory $u(t) = 1/(1 + exp(-\gamma t))$, with $\gamma = 50$, is required to be tracked by a 2nd order linear system (5), characterized by $\omega_n = 2$ rad/s, $l = 0{,}707$ and $k_3 = 0{,}7$.

**Figure** 6 shows the numerical results related to the 2nd order linear system for different $\gamma$ values, namely for 10, 20, 30, 40, 50 e 60. Optimized parameters of the BH controller (4): $p = q = 2$, $M_0 = 241.9686$, $k_1 = 0.8087$, $k_2 = 55.5633$, $r_0 = 0.2246$, $a = 6.0799$. Optimized parameters of the PID controller (6): $k_p = 356.9305$, $k_i = 2.9646$, $k_d = 0.7199$.

**Figure 7** shows experimental results related to the response of the BH controller (4) and PID controller (6) when a step trajectory is required to be tracked by a linear motion electromechanical servomechanism, actuated by a servomotor.

**Figure 8:** The Bardeen and Hayward BH correspond, respectively, to the choices p = 3, q= 2 and p=q= 3 in the mass function.

**Figure 9:** Closed-loop feedback control diagram incorporating the BH controller.

**Figure 10:** Algorithm flowchart used to obtain the numerical results.

**Figure 11:** Step response with BH controller to the 2nd order linear system.

**Figure 12:** Phase portrait of the system to the 2nd order linear system.

**Figure 13:** Output of a 2nd order system for parameters that result in instability.

**Figure 14:** Time response of all controlled systems for u given by (38) with $\gamma = 50$ (example 4).

**Figure 15:** Time response of all controlled systems to the 2nd order linear system for a unit ramp as reference (example 5).

**Figure 16:** (a) Relation between $\gamma$ and J for the discussed example 4, where a lower J value means a better performance; (b) Relation between the ramp slope and J for example 5; J axis is in logarithmic scale.

**Figure 17** shows an embodiment concept of cancer treatment using BH inspired dynamics.

**Figure 18** shows a schematic diagram of an embodiment, of the closed-loop feedback control incorporating a BH controller.

**Figure 19** shows an embodiment of optimization and response for multiple tumour dynamics.

**Figure 20** shows the results related to BH responses for the Cyclophosphamide drug.

**Figure 21** shows the results related to BH responses for the Atezolizumab drug.

## DETAILED DESCRIPTION

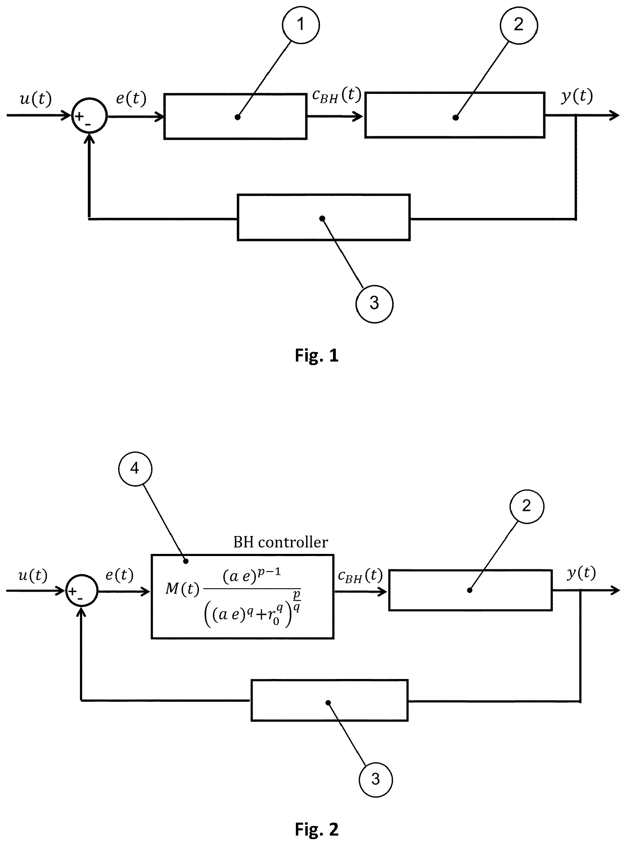

This invention concerns a non-linear control method, in which the controller response $c_{BH}$ is obtained by applying a control law formulated from models that describe one or more physical phenomena related and/or associated with Universal Gravitation. The overall control system is a closed-loop system with negative feedback, and it includes at least a controller (1), the dynamic system under control (2) and a sensing system (3), as shown in Figure 1. The reference $u$ is associated to a signal error e, obtained by the difference between the reference $u$ and the output $y$, which is monitored by the sensing system (3). This error is minimized by the controller (1) operation, through the application of a control signal $c_{BH}$ to the dynamic system under control (2). This is a control method with ability to track trajectories between different states of the system under control (2), or to ensure that required system state(s) are unchanged. The dynamic system under control may be linear or non-linear.

**Figures 1, 2, 3 and 4** schematically illustrate the block diagrams of the control system inspired by Black Hole dynamics, in which:

1 - generic controller based on Black Hole (BH) dynamics;
2 - dynamic system under control;
3 - sensing system;
4 - BH controller, whose control law is inspired by the Newtonian gravitational potential of regular Black Holes, according to the formulation proposed by Bardeen, and by the accretion of matter onto the Black Hole;
5 - 2nd order linear system;
6 - proportional, integral and derivative (PID) controller.

The block diagrams shown in Figures 1, 2, 3 and 4 refer to closed-loop systems with negative feedback, in which:

$u$ - reference to be tracked by the dynamic system under control (2) using BH controllers (1), (4) and PID (6);
$c_{BH}$ - control signal applied by BH controllers (1), (4) to the dynamic system under control (2), (5);
$c_{PID}$ - control signal applied by PID controllers (6) to the dynamic system under control (2);
$y$ - output of the dynamic system under control (2) in response to the control signals $c_{BH}$ or $c_{PID}$;
$e$ - error between $u$ and $y$;
$\omega_n$ - natural frequency of the 2nd order linear system under control (5);
$\xi$ - damping coefficient of the 2nd order linear system under control (5);
$k_3$ - gain of the 2nd order linear system under control (5);
$k_p$ - proportional gain of the PID controller (6);
$k_i$ - integral gain of the PID controller (6);
$k_d$ - derivative gain of the PID controller (6);

*t*-time.

The main goal of this control method is to allow the design of controllers (1) with superior performance: (i) without introducing intuition and lack of interpretability; (ii) with ability to be integrated in all natural environments where gravity can provide significant effects.

Numerical and experimental results confirm the ability of this controller (1) to achieve significant improvements in comparison to PID controllers when it is designed: (i) using the Newtonian gravitational potential of regular Black Holes, as formulated by Bardeen; and (ii) using the accretion of matter from the environment onto the Black Hole.

This control method can be applied to all technologies requiring trajectory tracking, such as in electric, hydraulic and pneumatic servomechanics, in chemical and thermofluid systems, and in biomedical systems. It can also be used as a tool in technologies related to the design and simulation of dynamic systems.

Considering the mentioned figures, a realization is here described in detail, which is not intended, however, to limit the scope of the present invention. The BH controller (1), exposed in Figure 1, can be implemented according to the control law

$$c_{BH}(t) = M(t) \frac{(ae)^{p-1}}{\left((ae)^q + r_0^q\right)^{p/q}}$$

with

$$M(t) = M_0 + k_1 exp\left(k_2\left(\int e\, dt\right)^2\right)$$

established from Newtonian gravitational potential of regular Black Holes, as formulated by Bardeen, and accretion of matter $M$, as illustrated in Figure 2. The following analogy was used: the reference $u$ represents the center of the Black Hole; the output $y$ of the dynamic system under control (2) is the position of a mass within the Black Hole (inside the Schwarzschild radius) over time, which must converge to the center $u$ of the Black Hole, or keep unchanged in this center $u$; this mass is negligible compared to the mass of the Black Hole; $r_0$ is a constant, formulated by Bardeen to ensure non-singular solution(s); the error $e = u - y$ is the distance (over time) between this mass and the center of the

Black Hole. $M_0$ is the initial mass of the Black Hole; $k_1$ and $k_2$ are parameters that define the accretion rate; $a, p \in \mathbb{N}^+$

and $q \in \mathbb{N}^+$ are parameters to define the gravitational potential. $c_{BN}$ must be at least of class $C^1$, and finite for any error value e. Therefore, $p$ and $q$ must be even: $p \in \{2k : k \in \mathbb{N}^+\}, q \in \{2k : k \in \mathbb{N}^+\}$ .

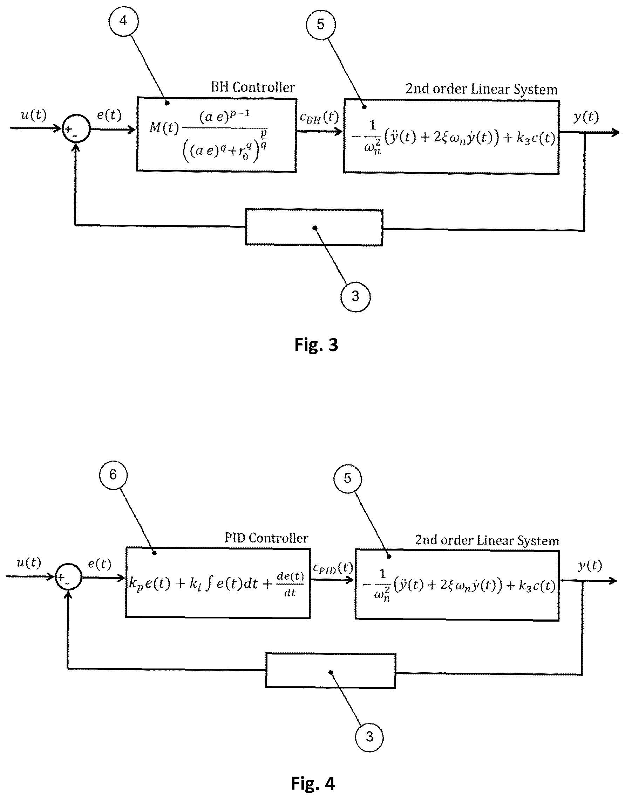

The controller (4) parameters must be optimized according to the type of system under control (2) and the performance requirements (maximum trajectory tracking error, maximum stationary error, maximum rise time, maximum settling time, etc.), namely the parameters $M_0$, $k_1$, $k_2$, $a$ and $r_0$. Such optimization can be carried out using hyperparameter tuning algorithms, such as the Nelder-Mead simplex algorithm or the BH optimization algorithm. The control of the dynamic system (2) must be performed in a closed loop with negative feedback, which includes, at least, the BH controller (4), the dynamic system under control (2), either linear (5) or non-linear, and a sensing system (3), which monitor the output $y$ over time $t$, as shown in Figures 2 and 3.

The performance of the BH controller (4) was compared to the PID controller (6) in the control of 2nd order linear systems (5)

$$\ddot{y}(t) + 2\xi\omega_n\dot{y}(t) + \omega_n^2 y(t) = k_3\omega_n^2 c(t)$$

where

$$c_{PID}(t) = k_p e(t) + k_i \int e(t)dt + \frac{de(t)}{dt}$$

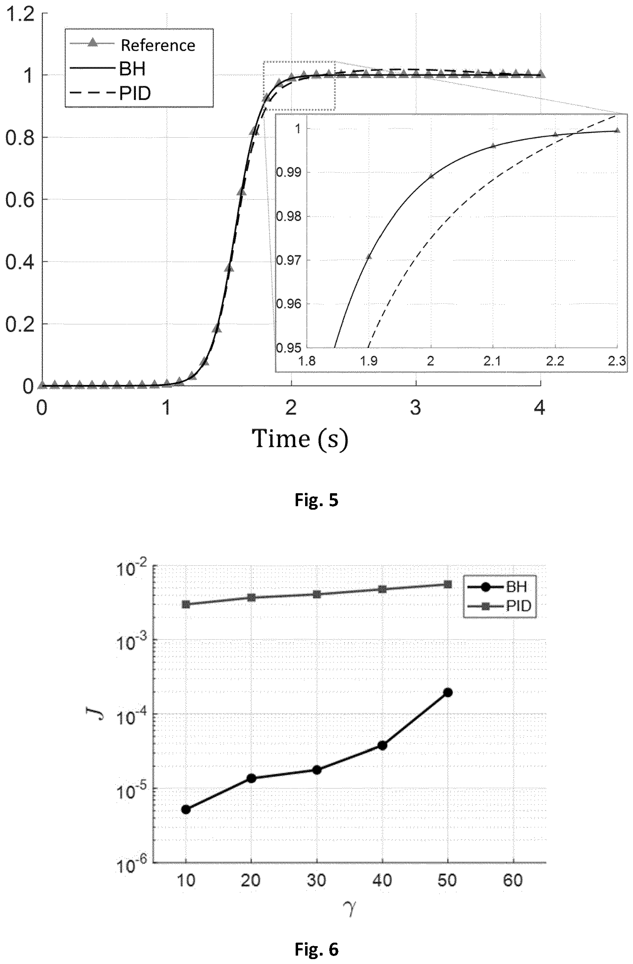

is the control signal of the PID controller (4). Numerical and experimental results highlight that the BH controller (4) can achieve improvements up to 100% in the tracking of sigmoid-logistic trajectories in comparison with the results obtained by the PID controller (6) in the control of 2nd order linear systems (5), as shown in Figures 5 and 6. These results were achieved by optimizing the controllers' parameters through the minimization of the function

$$J = \int_0^t \exp(\tau)\,(u - y)^2 d\tau$$

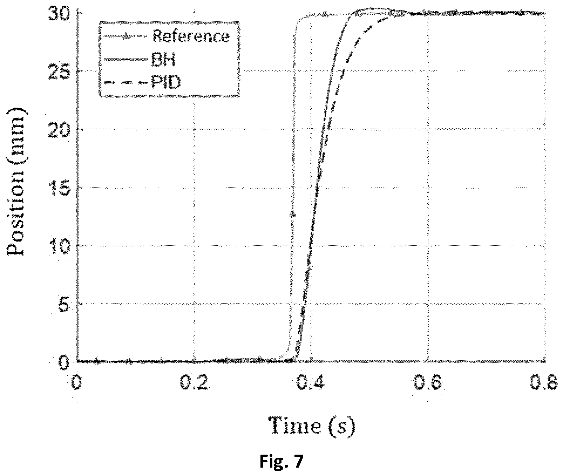

Numerical results expressed in Figure 6 show that errors lower or equal to $J_{BN}$ = 5.2 $\times$ 10$^{-6}$ were obtained by the BH controller (4) in the tracking of sigmoid-logistic trajectories, whereas $J_{PID}$ = 0.003 was obtained by the PID controller, which corresponds to 99.83% performance improvement. The experimental results exposed in Figure 7 shows performance improvements of 34.8% and 9.76% in the rising time (from 10% to 90% of the final reference $u$) and in the settling time (in the range below 2% of the final reference u).

The controller (1), (4) can operate using real-time hardware/software platforms, and requires a set of operations, of which the following ones must be highlighted:

(a) identify of the physical laws found in Black Holes that can be used to control dynamic systems;
(b) define the control law, which, by analogy, integrates the mathematical formulations used to describe the dynamics established in a);
(c) implement a controller (1) that applies control signals according to the control law established in b). The controller can be linearized at specific operating points of the dynamic system under control (2);
(d) integrate the controller (1), described in c), into a closed-loop structure with negative feedback.

In an embodiment, the control method establishes a control law based on mathematical formulations of physical dynamics found in Black Holes, to implement non-linear controllers (1), (4), which can operate into closed-loop structures with negative feedback that include, at least, a controller (1), the dynamic system under control (2) and a sensing system (3).

In an embodiment, control method comprises the following steps:

a) Identification of the physical laws found in Black Holes that can be used to control dynamic systems;
b) Specification of the control law, which, by analogy, integrates the mathematical formulations used to describe the dynamics established in a);
c) Implementation of a controller (1) that applies control signals according to the control law established in b);
d) Integration of the controller (1), described in c), into a closed-loop structure with negative feedback.

In an embodiment, the control method according to previous claims, characterized by establishing one or more control laws based on physical dynamics found in Black Holes.

**Figure 7** shows experimental results related to the response of the BH controller (4) and PID controller (6) when a step trajectory is required to be tracked by a linear motion electromechanical servomechanism, actuated by a servomotor, a system that can be approximately modelled by the transfer function $Y(s)/U(s)$ = (0.9633)(23.93)/(s(s + 23.93)). Optimized parameters of the BH controller (4): $p = q = 2$, $M_0$ = 2842.49, $k_1$ = 0.58, $k_2$ = 100.82, $r_0$ = 0.318, $a$ = 2.555. Optimized parameters of the PID controller (6): $k_p$ = 17.9148, $k_i$ = 1.13, $k_d$ = 0.025.

Since General Relativity is written in the language of differential geometry, so are its black hole solutions. They are described by a line element, $ds^2 = g_{\mu\nu}dx^\mu dx^\nu$, where $x^\mu$ are the coordinates covering the spacetime, with $x^\mu = (ct,x^i)$, $t$ is the time coordinate, c is the velocity of light and $x^i$ are the spatial coordinates, with $i$ = 1,2,3. The line element is defined by the metric components $g_{\mu\nu}$, a 4 $\times$ 4 symmetric matrix whose entries are spacetime functions. In accordance to standard General Relativity literature, it is herein used geometrised units, with Newton's constant G and the speed of light c, taken to obey $G = 1 = c$. Mathematically, it determines how infinitesimal displacements in an arbitrary chart covering (locally) the spacetime manifold, relate to the infinitesimal spacetime interval $ds$ along a curve. Physically, the metric components are gravitational potentials. They generalise to the relativistic realm the Newtonian gravitational potential $\Phi$, that completely determines the Newtonian gravitational field. In the Newtonian limit of General Relativity, the temporal-temporal metric component $g_{tt}$ is related to the Newtonian potential as $g_{tt}$ = -(1 + 2$\Phi$) (1).

In an embodiment, it is focused on spherically symmetric regular black holes, which are described by a diagonal metric, with a line element of the form

$$ds^2 = -f(r)dt^2 + \frac{\{dr^2\}}{\{f(r)\}} + r^2 d\,\Omega_2 \,(2)$$

, where $(d\Omega_2 = d\theta^2 + sin^{2\theta}d\phi^2)$ is the line element on the unit 2-sphere, and

$$f(r) = 1 - \frac{2m(r)}{r}$$

, where m(r) is the so-called mass function. For $m(r) = M$ = constant, this is the Schwarzschild solution of vacuum General Relativity. As a more general choice, taking m(r) as

$$m(r) = \frac{M_0}{\left(1 + \left(\frac{r_0}{r}\right)^q\right)^{\frac{p}{q}}} \,, \qquad (3)$$

still guarantees an asymptotically flat spacetime for positive $p$ and $q$. $M_0$ and $r_0$ are, respectively, a mass and a length parameters. For $p = 3$ and $q = 2$, Eq. 2 becomes the Bardeen regular black hole, a pioneering example of this type of configurations. As another example, for $p = 3$ and $q = 3$, Eq. 2 becomes the Hayward regular black hole (Fig. 8).

**Figure 8:** The Bardeen and Hayward BH correspond, respectively, to the choices $p = 3$, $q = 2$ and $p = q = 3$ in the mass function.

The strategy herein is to extract the corresponding Newtonian potential of these regular black holes, via Eq. 1. For the model (2) with (3), one obtains

$$\Phi = -\frac{M_0 \, r^{p-1}}{\left(r^q + r_0^q\right)^{\frac{p}{q}}} \,. \qquad (4)$$

Strictly speaking, Eq. 1 is only a valid relation for $\Phi \ll 1$. Thus, the identification in (4) is taken as a Newtonian potential inspired by black hole physics, rather than derived from it.

Whereas the line element (2), with choices such as (3), describes a static black hole solution (not evolving in time), in a real astrophysical process, the mass of a BH is not constant, due to accretion of matter from the environment onto the black hole. The morphology of the accretion flow is mainly determined by the angular momentum of the accreting gas, and the mass accretion rate.

The accretion of mass by a black hole is also a phenomenon that provided grounds for developing the control method proposed in this document.

Gravitation is one of the fundamental interactions, which effects are experienced in most environments where a control method is required.

Analyses are focused on its ability to control general second order linear systems. Besides, such new BH controllers can be integrated in all natural environments experiencing gravity, such that the amount of energy required to perform specific trajectories can be minimised.

Let's consider a generic second order system, which dynamics are described by

$$\begin{cases} \dot{\mathbf{x}} = \mathbf{g}(t, \mathbf{x}, \mathbf{u}) \\ \mathbf{y} = \mathbf{h}(t, \mathbf{x}) \end{cases} , \qquad (5)$$

with

$$\mathbf{g}(t, \mathbf{x}, \mathbf{u}) = \begin{cases} \dot{x}_1 = x_2 \\ \dot{x}_2 = -\omega_n^2 x_1 - 2\xi\omega_n x_2 + k_1 u \end{cases} , \qquad (6)$$

where (t) is the time variable, $x$ is the state vector, $u$ is the input vector, $\xi$ is the damping factor, $\omega_n$ is the natural frequency and $k_1$ is the system gain.

**Assumption 1:** It is assumed that the system to be controlled is linear. Therefore, ($\xi > 0$), ($\omega_n > 0$) and (5) can be written in the form

$$\begin{cases} \dot{x} = Ax + Bu \\ y = Cx \end{cases} . \qquad (7)$$

**Assumption 2:** It is assumed that C = $I$, where I is the identity matrix, which means that $y = x$ so $y$ can be accurately measured.

**Assumption 3:** It is assumed that the system (5) is controllable, which means that $rank(R) = n$, where $R = [B\ AB\ A^2B\ ...A^{n-1}B]$) is the controllability matrix and $n$ is the system order. For such reason, $k_1$ is limited to be strictly positive.

The present control method is capable to solve a trajectory tracking problem when operating in a feedback-loop based scheme, which consists in generating a control signal $c$, such that the output $y$, tracks the desired trajectory $u$.

The proposed controller has parameters that can be tuned and optimised, minimising the tracking error $e$ using the fitting function

$$J = \int_0^t \exp(t)\,(u - y)^2 dt . \qquad (8)$$

**Figure 9:** Closed-loop feedback control diagram incorporating the BH controller.

Let $\varepsilon$ be a sufficiently small value such that $\varepsilon \gtrsim 0 \sim (\varepsilon \neq 0)$, and $\delta$ is defined as

$$\delta = \varepsilon\,\mathrm{sign}(u), \qquad (9)$$
,

such that the proposed control diagram is defined as illustrated by Fig. 9.

As stated in (4), the gravitational potential is function of the distance $r$ between a point in space and the BH centre.

The control law mimics the gravitational potential according to a set of analogies:

the reference $u$, represents the centre point of the BH, the control signal $c_{BH}$, or $c$, is the gravitational potential, the output $y$ is the position of the mass that is attracted by the BH, and, therefore, the error $e$ is the distance between the mass and the BH centre (Table 1), defined as $e = u - y$ (10).

<div align="center">

**Table 1:** Control Systems vs. Black Hole analogies

| Control Systems | Black Hole System |
|---|---|
| Reference ($u$) | BH centre |
| Control signal ($c$) | Gravitational potential |
| Output ($y$) | Position of the mass |
| Error ($c$) | Distance between the mass and the BH centre |

</div>

Since it is assumed that $y = x_1$, the control law is defined as

$$c(t, \mathbf{x}) = M(t, \mathbf{x})\,\frac{(ae)^{p-1}}{((ae)^q + r_0^q)^{\frac{p}{q}}} , \qquad (11)$$

where $p \in \mathbb{N}^+$ and $q \in \mathbb{N}^+$ .

The BH mass M is defined as

$$M(t, \mathbf{x}) = M_0 + k_2 \exp\left( k_3 \left( \int_0^t e\,dt \right)^2 \right) , \qquad (12)$$

where $M_0$ is the initial mass of the BH, and $k_2$ and $k_3$ are parameters that may be tuned defining the accretion rate.

The function $c(t,x)$ must be (at least) of class $C^1$, odd and finite for any error value at constant mass. Therefore, some parameters must be constrained to ensure such properties.

Parameters $p$ and $q$ should be chosen such that the control function does not have singularities. Possible singularities may arise when

$$((ax)^q + r_0^q)^{\frac{p}{q}} = 0 \ . \tag{13}$$

If $q$ is odd, Eq. (13) has real solution, and, therefore, the control function $c$ is singular, which must not occur, as the same c is required $\forall e$. However, if $q$ is even, Eq. (13) has no real solution, and, therefore, no singularity in the control function is found. Thus, $q \in \{2k: k \in N^+\}$).

The control $c(x)$ will be an odd function at constant mass if $-c(x) = c(-x)$ holds for all $x$.

Since $q \in \{2k: k \in N^+\}$,

$$((ax)^q + r_0^q)^{\frac{p}{q}} = ((a(-x))^q + r_0^q)^{\frac{p}{q}} \ , \tag{14}$$

which shows that the denominator of the control function is even.

The quotient between an even function and an odd function is an odd function.

Thus, for c to be odd, $(ax)^{p-1}$ must be odd. To satisfy this condition, $p \in \{2k: k \in N^+\}$.

The control function $c$ must be at least from class $C^1$. The first derivative of $c$ is

$$\frac{d}{dx}c(x) = \frac{(p-1)(ax)^{p-2} - p(ax)^{p+q-2}((ax)^q + r_0^q)^{-1}}{((ax)^q + r_0^q)^{\frac{p}{q}}} \ , \tag{15}$$

which is continuous and, therefore, it is always of class $C^1$.

Since the controller introduces an integral, the closed-loop system is a third order system.

A new state variable, $z$, is then needed. The closed-loop system is given by

$$\dot{\mathbf{x}} = \mathbf{f}(t, \mathbf{x}) \Leftrightarrow$$

$$\begin{cases} \dot{x}_1 = x_2 \\ \dot{x}_2 = -\omega_n^2 x_1 - 2\xi\omega_n x_2 + k_1 \left[ M_0 + k_2 e^{k_3 z^2} \right] \frac{(a(u-x_1))^{p-1}}{\left((a(u-x_1))^q + r_0^q\right)^{\frac{p}{q}}} \\ \dot{z} = u - x_1 - \delta \end{cases} \tag{16}$$

**Theorem IV.1:** Let's consider the function $f(t,x)$ with $x \in R^n$. A dynamical system $x = f(t,x)$ given by (16), for which Assumptions 1, 2 and 3 hold, has a unique solution for the initial condition $x(t_0) = x_0$ and for $t \in [t_0, t1])$ if:

1. $f(t, x)$ is piece-wise continuous $\forall t \in [t_0, t_1]$; and
2. $f(t, x)$ is global Lipschitz $\forall t \in [t_0, t_1]$;

Consider that

$$\mathbf{f}(t, \mathbf{x}) = \begin{bmatrix} f_1(t, \mathbf{x}) \\ f_2(t, \mathbf{x}) \\ f_3(t, \mathbf{x}) \end{bmatrix} \ . \tag{17}$$

Function $f_1(t, x)$ is affine so is continuous; Function $f_2(t, x)$ is obtained by the composition of sums, multiplications and powers, and therefore the result is a continuous function since the individual terms are continuous; Function $f_3(t, x)$ is affine so is continuous.

The Jacobian matrix of $f(t, x)$, $J_f$ is

$$J_f = \begin{bmatrix} 0 & 1 & 0 \\ \frac{\partial f_2}{\partial x_1} & -2\xi\omega_n & \frac{\partial f_2}{\partial z} \\ -1 & 0 & 0 \end{bmatrix}, \tag{18}$$

where:

$$\frac{\partial f_2}{\partial x_1} = -\omega_n^2 + k_1 \left[ M_0 + k_2 \exp\left(k_3 z^2\right) \right] \frac{a^{p-1}(u - x_1)^{p-2})}{((a(u - x_1))^q + r_0^q)^{\frac{p}{q}}} \left[ 1 - p + \frac{pa^q(u - x_1)^q}{(a(u - x_1))^q + r_0^q} \right] \tag{19}$$

$$\frac{\partial f_2}{\partial z} = 2k_1 k_2 k_3 z \exp\left(k_3 z^2\right) \frac{(a(u - x_1))^{p-1}}{((a(u - x_1))^q + r_0^q)^{\frac{p}{q}}}. \tag{20}$$

According to (18) all the partial derivatives of $f(t, x)$ are continuous and bounded ($\forall t \in [t_0, t_1]$). Thus, as $f(t, x)$ is continuous and its partial derivatives $\frac{\partial}{\partial x_i} f_j$ are continuous and globally bounded for all $x \in R^n$, then $f(t, x)$ is globally Lipschitz in $[t_0, t_1]$.

If $f(t, x)$ is continuous, then it is also piece-wise continuous. Since $f(t, x)$ is globally Lipschitz and also piece-wise continuous, then, $\dot{x} = f(t, x)$ has a unique solution for ($t \in [t_0, t_1]$) for each set of initial conditions $x_0$, and for each reference $u$.

The point $x^* = [x_1\ x_2\ z]^T$, $x_1, x_2, z \in R$, where

$$x_1 = u - \delta ,$$

$$x_2 = 0 ,$$

$$z = \pm\sqrt{\frac{1}{k_3} \ln\left(\frac{((a\delta)^q + r_0^q)^{\frac{p}{q}}}{(a\delta)^{p-1}} \frac{\omega_n^2}{k_1 k_2}(u - \delta) - \frac{M_0}{k_2}\right)}, \tag{21}$$

is a equilibrium point of system (16).

This result is achieved solving

$$\mathbf{f}(t, \mathbf{x}) = 0 , \tag{22}$$

The signal z depends on the value given by $\int_{t_0}^{t_1}(e - \delta)dt$.

Theorem IV.2: For sufficiently small values of $\varepsilon$, the condition

$$- 2\xi\omega_n^3 \left[ -1 + (u - \delta) \left( \frac{ap(a\delta)^{q-1}}{(a\delta)^q + r_0^q} + \frac{1-p}{\delta} \right) \right]$$

$$> 2k_1 k_2 k_3 vw \left( \pm\sqrt{\frac{\ln(w)}{k_3}} \right) ,$$

is sufficient to determine the stability of the equilibrium point $x^*$ of the system given by (16).

Equilibrium points can be classified depending on the signs of the eigenvalues of the linearization about the equilibrium point, which is equivalent to evaluate the Jacobian matrix at each of the equilibrium points of (16), and then finding the resulting eigenvalues.

The eigenvalues can be founded solving the following equation:

$$\det\left(J - \lambda I\right)\big|_{x=x^*} = 0 , \tag{23}$$

which is equivalent to

$$\lambda^3 + 2\xi\omega_n\lambda^2 - \frac{\partial f_2}{\partial x_1}\bigg|_{x=x^*} \lambda + \frac{\partial f_2}{\partial z}\bigg|_{x=x^*} = 0 . \tag{24}$$

An equilibrium point is stable if the real part of all the roots of Eq. (24) is less than zero. The Routh-Hurwitz criterion states that a third-order polynomial $P(s) = s^3 + a_2 s^2 + a_1 s + a_0$ has all roots in the left half-plane if and only if $a_0$, $a_1$ and $a_2$ are positive, and $a_2 a_1 > a_0$. Thus, the system (16) will be stable if

1)

$$2\xi\omega_n > 0 ; \tag{25}$$

2)

$$- \frac{\partial f_2}{\partial x_1}\bigg|_{x=x^*} > 0 ; \tag{26}$$

3)

$$\frac{\partial f_2}{\partial z}\bigg|_{x=x^*} > 0 ; \tag{27}$$

4)

$$- 2\xi\omega_n \frac{\partial f_2}{\partial x_1}\bigg|_{x=x^*} > \frac{\partial f_2}{\partial z}\bigg|_{x=x^*} . \tag{28}$$

Consider $v$ and $w$ such that

$$v := \frac{(a\delta)^{p-1}}{((a\delta)^q + r_0^q)^{\frac{p}{q}}} , \tag{29}$$

$$w := \left( \frac{((a\delta)^q + r_0^q)^{\frac{p}{q}}}{(a\delta)^{p-1}} \frac{\omega_n^2}{k_1 k_2}(u - \delta) - \frac{M_0}{k_2} \right) . \tag{30}$$

The inequalities (25), (26) and (27) must be analysed by finding the limit when $\varepsilon \to 0$. Since $\delta = \varepsilon$ sign(u), when $\varepsilon \to 0$, $\delta \to 0^{\pm}$ depending on *sign(u)*. Thus,

$$\lim_{\delta \to 0^{\pm}} v = 0^{\pm} \tag{31}$$

and

$$\lim_{\delta \to 0^{\pm}} w = +\infty . \tag{32}$$

By Assumption 1, $\xi > 0$ and $\omega_n > 0$, which implies that $2\xi\omega_n > 0$ holds $\forall \delta, x$.

Concerning the condition (26), when $\varepsilon$ is sufficiently small,

$$\lim_{\varepsilon \to 0} \left( -\left. \frac{\partial f_2}{\partial x_1} \right|_{x=x^*} \right)$$
$$= \lim_{\varepsilon \to 0} \left( \omega_n^2 - \omega_n^2(u - \delta) \left[ \frac{1-p}{\delta} + \frac{pa^q \delta^{q-1}}{(a\delta)^q + r_0^q} \right] \right) \tag{33}$$
$$= \omega_n^2 - \omega_n^2 u(\mp\infty) > 0 .$$

Concerning the condition (27):

$$\lim_{\varepsilon \to 0} \left( \left. \frac{\partial f_2}{\partial z} \right|_{x=x^*} \right)$$
$$= \lim_{\varepsilon \to 0} \left( 2k_1 k_2 k_3 \left( \pm\sqrt{\frac{1}{k_3} \ln w} \right) \left[ \frac{\omega_n^2}{k_1 k_2}(u - \delta) - v\frac{M_0}{k_2} \right] \right) \tag{34}$$
$$= (\pm\infty) \left[ \frac{\omega_n^2}{k_1 k_2} u \right] > 0 ,$$

which is always true for sufficiently small values of $\varepsilon$, since the positive solution of z corresponds to positive *u* values, and vice-versa.

Since the inequalities (25), (26) and (27) are always satisfied for small $\varepsilon$ values, then, system stability only depends on condition (28), which can be simplified as written in the following steps:

$$-2\xi\omega_n \left.\frac{\partial f_2}{\partial x_1}\right|_{x=x^*} > \left.\frac{\partial f_2}{\partial z}\right|_{x=x^*}$$

$$\Leftrightarrow -2\xi\omega_n \left[ -\omega_n^2 + k_1 \left( M_0 + k_2 \left( \frac{\omega_n^2}{vk_1k_2}(u-\delta) - \frac{M_0}{k_2} \right) \right) \right.$$
$$\left. \frac{v}{\delta} \left( \frac{p(a\delta)^q}{(a\delta)^q + r_0^q} + 1 - p \right) \right]$$
$$> 2k_1k_2k_3vw \left( \pm\sqrt{\frac{\ln(w)}{k_3}} \right)$$

$$\Leftrightarrow -2\xi\omega_n \left[ -\omega_n^2 + \omega_n^2(u-\delta) \left( \frac{1}{\delta}\frac{p(a\delta)^q}{(a\delta)^q + r_0^q} + \frac{1-p}{\delta} \right) \right]$$
$$> 2k_1k_2k_3vw \left( \pm\sqrt{\frac{\ln(w)}{k_3}} \right)$$

$$\Leftrightarrow -2\xi\omega_n^3 \left[ -1 + (u-\delta) \left( \frac{ap(a\delta)^{q-1}}{(a\delta)^q + r_0^q} + \frac{1-p}{\delta} \right) \right]$$
$$> 2k_1k_2k_3vw \left( \pm\sqrt{\frac{\ln(w)}{k_3}} \right) \quad , \quad (35)$$

where the $\pm$ signal depends on $\int_{t_0}^{t_1}(e-\delta)dt$ value. The proof is complete.

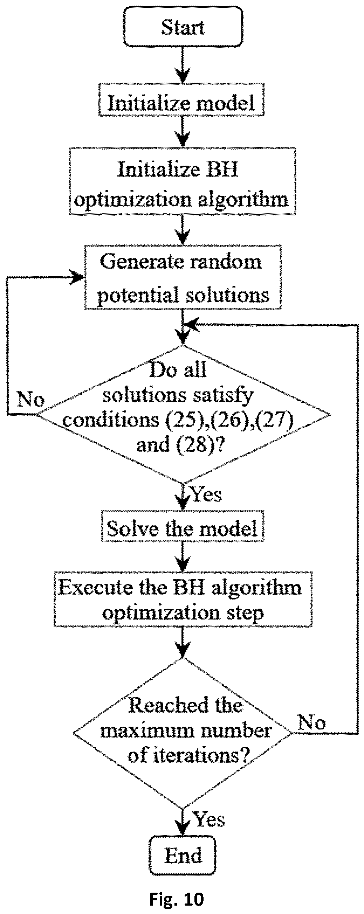

Various numerical simulations, here presented, were performed to highlight strong evidences related to the theoretical results, as well as to present some performance results achievable by the BH controller here reported. The simulation process was performed as described in Fig. 10.

**Figure 10:** Algorithm flowchart used to simulate the numerical results.

The optimal parameters that minimises (8) were achieved using BH optimisation method. The values used for all the parameters in numerical simulations are presented in Table 2.

**Table 2:** Values of both model and controller parameters used in all numeric examples.

The parameters $k_p$, $k_i$ and $k_d$ are the constant gains of PI and PID controllers. For all the examples $\varepsilon = 1 \times 10^{-9}$.

| Example # | Controller Type | Model Parameters | | | | | | | | Controller Parameters | | |
|---|---|---|---|---|---|---|---|---|---|---|---|---|
| | | $\omega_n$ | $\xi$ | $k_1$ | $M_0$ | $k_2$ | $k_3$ | $r_0$ | $a$ | $k_p$ | $k_i$ | $k_d$ |
| Example 1 | BHp2 | 2 | 1.2 | 0.7 | 250.4662 | 0.2351 | 37.3277 | 0.2295 | 2.0605 | - | - | - |
| Example 2 | BHp2 | 2 | 2 | 1 | 467.3290 | 0.3257 | 45.4403 | 0.4213 | 2.7609 | - | - | - |
| Example 3 | BHp2 | 2 | 1.2 | 0.7 | 250.4662 | 0.2351 | $6.41 \times 10^{17}$ | 0.2295 | 2.0605 | - | - | - |
| Example 4 | BHp2 | 2 | 0.707 | 0.7 | 241.9686 | 0.8087 | 55.4633 | 0.2246 | 6.0799 | - | - | - |
| | BHp4 | 2 | 0.707 | 0.7 | 342.2999 | 0.0807 | 80.3339 | 0.157 | 6.3687 | - | - | - |
| | PI | 2 | 0.707 | 0.7 | - | - | - | - | - | 448.7791 | 0.029 | - |
| | PID | 2 | 0.707 | 0.7 | - | - | - | - | - | 356.9305 | 2.9646 | 0.7199 |
| Example 5 | BHp2 | 2 | 0.707 | 0.7 | 343.4724 | 0.1837 | 17.3925 | 0.1499 | 6.7006 | - | - | - |
| | BHp4 | 2 | 0.707 | 0.7 | 277.9455 | 0.395 | 60.0774 | 0.2363 | 5.7187 | - | - | - |
| | PI | 2 | 0.707 | 0.7 | - | - | - | - | - | 368.5147 | 0.9896 | - |
| | PID | 2 | 0.707 | 0.7 | - | - | - | - | - | 390.6477 | 9.355 | 0.2749 |

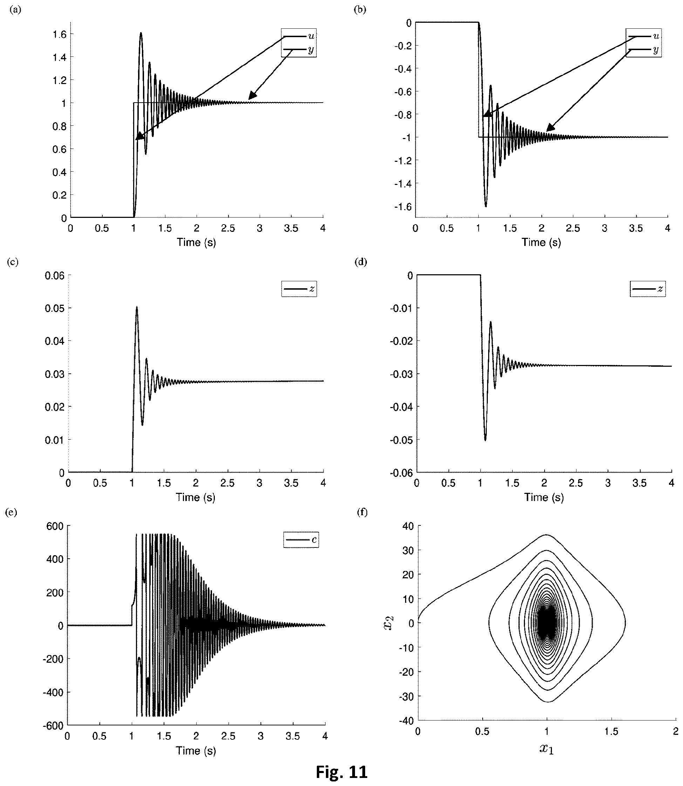

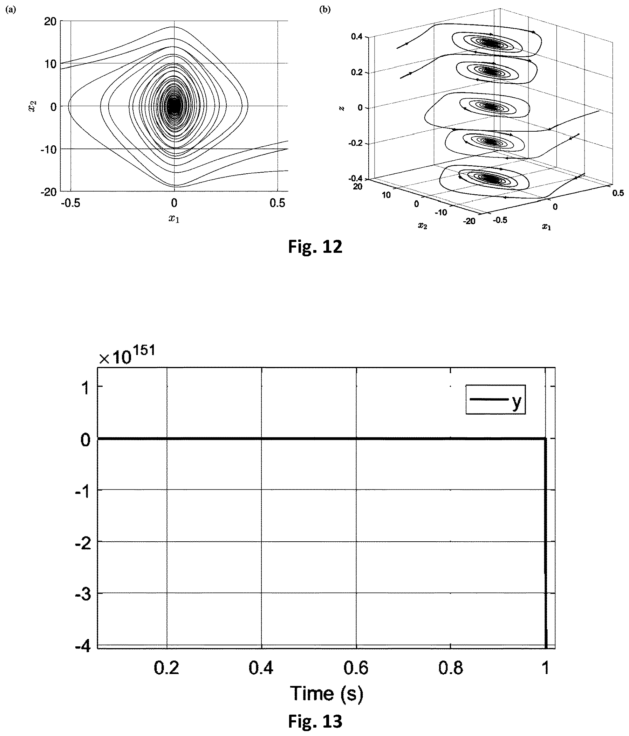

**Figure 11:** Step response with BH controller. (a) Step response for a positive unit step; (b) Step response for a negative unit step; (c) Plot of the state variable $z(t)$ corresponding to the positive unit step of Fig. 12a; (d) Plot of the state variable $z(t)$ corresponding to the negative unit step of Fig. 12b; (e) Control signal $c(t)$ corresponding to the Fig. 12a output; (f) Plot of the curve $(x_1(t), x_2(t))$ corresponding to the Fig. 12a output. The initial condition was $x_1 =$, $x_2 =$ and $z = 0$.

The first simulation refers to a trajectory tracking problem where the desired trajectory $u$ is a unit step. Two different step responses were analysed, one for a positive step and another one for a negative step.

When solving Eq. (22), due to the presence of a $z^2$ term, (21) has two solutions, a positive and a negative one. Nevertheless, the signal of the solution is dependent on $\int_{t_0}^{t_1}(e - \delta)dt$.

Fig. 11 shows that for $x(t_0) = [0\,0\,0]^T$, $\int_{t_0}^{t_1}(e - \delta)dt$ is positive for a positive step response (Fig. 11a), and, therefore, $z$ has a positive solution (Fig. 11c); the vice versa behaviour occur for the negative step (Fig. 11b,d).

Regarding $c(t)$, notice that the control signal is maximum around the equilibrium point, but decreases as close as the output approaches it (Fig. 11e), which expresses the fundamental dynamics shown in Fig. 8.

Observing Fig. 12f, one can also observe that the response oscillation frequency increases as the output approaches the desired trajectory, describing a spiral trajectory towards a centre point corresponding to the equilibrium point $x^* = [x_1 \, x_2 \, z]^T \cong [1\ 0\ 0.0278]^T$. The rise time of the system from 10% to 90% was 0.0451 s and the settling time (2%) was 1.2497 s, with an overshoot of 60.79%.

A regulation problem for null input reference, was solved for multiple sets of initial conditions.

A phase portrait of the system (16) was obtained (Fig. 12). For each initial condition, the related spiral trajectory shows a stable centred equilibrium point of the form $x^* = [x_1 \, x_2 \, z]^T \cong [0\ 0\ z]^T$ (Fig. 12a), where $z$ differs for each trajectory (Fig. 12b), meaning that the state variable $z$ is dependent on the initial condition. Trajectory patterns further suggest the nonlinear nature of the system.

**Figure 12:** Phase portrait of the system. Phase portrait where each line corresponds to a different initial condition; (a) 2D view of phase portrait; (b) 3D view of phase portrait.

This example is intended to illustrate a scenario where the condition (28) is violated. The inequality (28) can be rearranged as

$$-2\xi\omega_n \left.\frac{\partial f_2}{\partial x_1}\right|_{x=x^*} > \left.\frac{\partial f_2}{\partial z}\right|_{x=x^*} \tag{36}$$

$$\Leftrightarrow -2\xi\omega_n \left.\frac{\partial f_2}{\partial x_1}\right|_{x=x^*} - \left.\frac{\partial f_2}{\partial z}\right|_{x=x^*} > 0 \;.$$

Taken the parameters stated in Table 3, it is obtained

$$-2\xi\omega_n \left.\frac{\partial f_2}{\partial x_1}\right|_{x=x^*} - \left.\frac{\partial f_2}{\partial z}\right|_{x=x^*} = -2.05 \times 10^7 < 0 \;, \tag{37}$$

which exhibits the system instability. The system instability is shown in Fig. 13, which allows to observe the system output $y$ going to $(-)\infty$ after a step excitation ( $\lim_{t\to\infty} y(t) = \infty$ ).

**Figure 13:** Output of a second order system for parameters that result in instability.

In an embodiment, a desired trajectory logistic-sigmoid curve, defined as

$$u(t) = \frac{1}{1 + \exp\left(-\gamma(t - \tau)\right)} \;, \tag{38}$$

was used as the input excitation, where $\gamma$ defines the slope and $\tau$ a time shift. The maximum slope for each tested $\gamma$ is

shown in Table 3.

**Table 3**: Maximum slope for a logistic function trajectory as a function of $\gamma$. Note that the maximum slope of this curve is $\frac{\gamma}{4}$.

| $\gamma$ | Maximum slope |
|----|----|
| 10 | 2.5 |
| 20 | 5 |
| 30 | 7.5 |
| 40 | 10 |
| 50 | 12.5 |

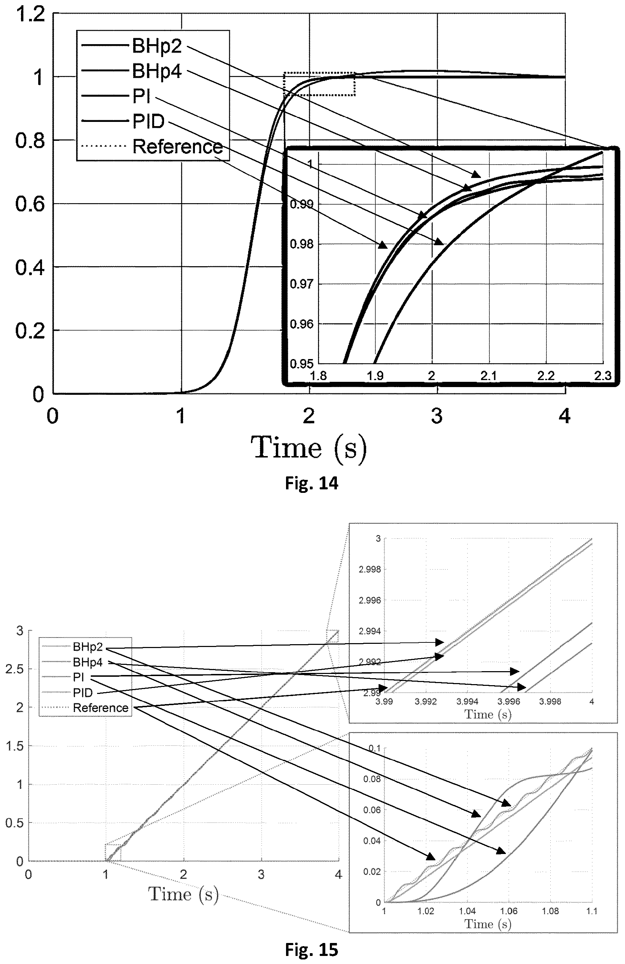

The performances of BHp2, BHp4, PI and PID controllers were analysed (BHp2 and BHp4 are the proposed Black Hole controller with $p = 2$ and $p = 4$, respectively). The results for $\gamma = 50$ may be observed in Fig. 14. The maximum error achieved by the BHp2 was $1.92 \times 10^{-4}$, while 0.028 was provided by the PID, which correspond to an improvement of 99.3%. Concerning the maximum time delay related to the reference signal, 0.0049s for BHp2 and 0.108s for PID were found, which corresponds to a relevant improvement of 95.4%.

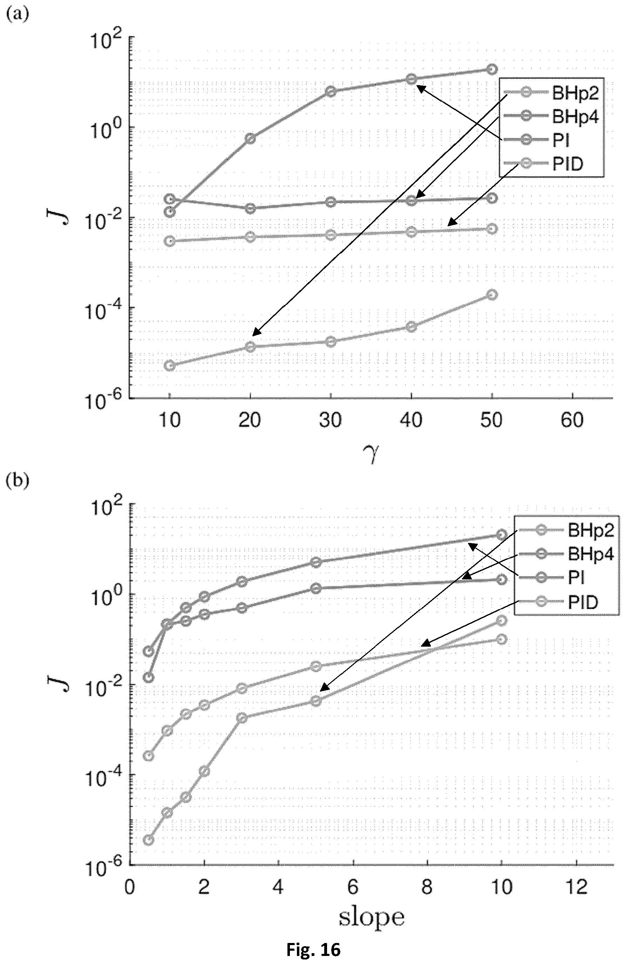

Concerning the best scenario $\gamma = 10$, $J = 0.003$ was found for PID and $J = 5.20 \times 10^{-6}$ for the BHp2, which represents a meaningful improvement of 99.83 %. Nevertheless, slightly $J$ decreases occur as $\gamma$ increases. as highlight in Fig. 16.

**Figure 14:** Time response of all controlled systems for $u$ given by (38) with $y = 50$ (example 4).

In an embodiment, ramp responses under BHp2, BHp4, PI and PID control were analysed for slopes ranging from 0.5 to 10.

Errors up to 0.0019 and 0.0068 were respectively found for the BHp2 and PID controllers (Fig. 8), which express an improvement of 72.5%. Concerning the maximum delay, 0.0019 s and 0.0068s were respectively achieved for BHp2 and PID, representing an improvement of 72.4%. Concerning the best case (slope = 0.5), the PID can provide $J = 2.62 \times 10^{-4}$, whereas the BHp2 is able to provide $J = 3.58 \times 10^{-6}$, which represents a significant improvement of 98.63 %.

**Figure 15:** Time response of all controlled systems for a unit ramp as reference (example 5).

Although the proposed BH controller with $p = 2$ outperforms the PID controller for step and ramp responses, the BH controller with $p = 4$ is only able to outperform the PI controller.

Table IV shows a more complete performance comparison between BHp2 and PID for the results obtained in examples 4 and 5.

**Table IV:** Performance of BH controller with p=2 and PID for all the simulated scenarios (example 4 and 5).

| | | $J_{BHp2}$ | $J_{PID}$ | Improv.[a] (%) |
|---|---|---|---|---|
| Example 4 | $\gamma = 10$ | $5.20 \times 10^{-6}$ | $3 \times 10^{-3}$ | 99.83 |
| | $\gamma = 20$ | $1.37 \times 10^{-5}$ | $3.7 \times 10^{-3}$ | 99.63 |
| | $\gamma = 30$ | $1.78 \times 10^{-5}$ | $4.1 \times 10^{-3}$ | 99.57 |
| | $\gamma = 40$ | $3.78 \times 10^{-5}$ | $4.8 \times 10^{-3}$ | 99.21 |
| | $\gamma = 50$ | $1.96 \times 10^{-4}$ | $5.6 \times 10^{-3}$ | 96.5 |
| Example 5 | $slp.^{b} = 0.5$ | $3.58 \times 10^{-6}$ | $2.62 \times 10^{-4}$ | 98.63 |
| | $slp. = 1$ | $1.45 \times 10^{-5}$ | $9.45 \times 10^{-4}$ | 98.47 |
| | $slp. = 1.5$ | $3.2 \times 10^{-5}$ | $2.22 \times 10^{-3}$ | 98.56 |
| | $slp. = 2$ | $1.19 \times 10^{-4}$ | $3.5 \times 10^{-3}$ | 96.6 |
| | $slp. = 3$ | $1.8 \times 10^{-3}$ | $8.2 \times 10^{-3}$ | 78.05 |
| | $slp. = 5$ | $4.3 \times 10^{-3}$ | $2.5 \times 10^{-2}$ | 82.8 |
| | $slp. = 10$ | 0.26 | 0.1 | -160 |

[a] Improv. is abbreviation of Improvement.
[b] slp. is abbreviation of slope.

**Figure 16:** (a) Relation between $\gamma$ and J for example 4, where a lower J value means a better performance; (b) Relation between the ramp slope and J for example 5; J axis is in logarithmic scale.

In an embodiment, the control method establishes one or more controllers (1), linearized at specific operating points of the dynamic system under control (2).

In an embodiment, the control method establishes the control operation by using real-time hardware/software platforms.

For the case of cancer therapy, the present disclosure establishes a survival trapped paradigm, in which patients (those selected during the screening process) do not know exactly how much time the treatment will take, but they know they will survive.

This disclosure establishes a bridge between Black Hole Physics and real-world cancer treatment, as a free-cancer state or local tumour retention states can be achieved defining attraction dynamics according to the proposed physical laws expressing how masses are attracted to BHs. Such astrophysical phenomenon ensures a trapped region (e.g., within the event horizon or Schwarzschild radius) in spacetime, in which matter cannot escape from it, and it is destroyed as it approaches the BH centre.

**Figure 17** shows an embodiment concept of cancer treatment using BH inspired dynamics: a, BH inspired scenario, in which tumour masses are attracted to its centre, such that they can be trapped and/or destroyed; b, Schematic diagram of the BH-tumour system within the cancer event horizon (cancer-adapted Schwarzschild radius); c, concept of chemotherapy controlled by BH dynamics.

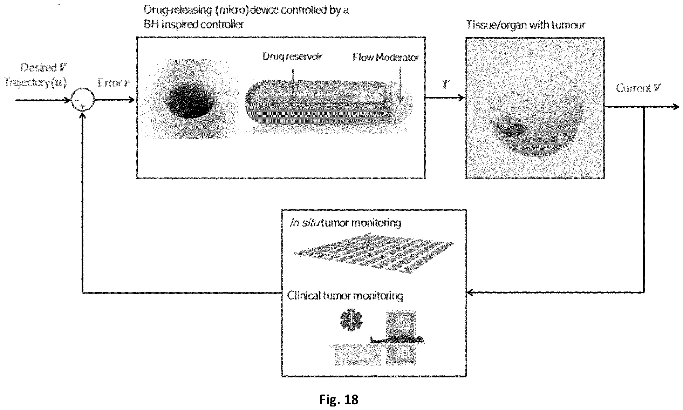

**Figure 18** shows a schematic diagram of an embodiment of the closed-loop feedback control incorporating a BH controller.

In an embodiment, two laws (the following equations) were used to design a BH controller:

(i) The Newtonian gravitational potential Φ of regular Black Holes, as formulated by Bardeen,

$$\Phi = \frac{M\,(k_2\,r)^{p-1}}{\left((k_3\,r)^q + r_0^q\right)^{\frac{p}{q}}} \,, \tag{101}$$

where $M \in \mathbb{R}^+$ is the BH mass, $r \in \mathbb{R}^+$ is the distance between the mass and the BH centre (Fig. 8b),

$r_0 \in \mathbb{R}^+$ is a parameter that ensures non-singular solutions; $p \in \mathbb{N}^+, q \in \mathbb{N}^+, k_2 \in \mathbb{R}^+$ and $k_3 \in \mathbb{R}^+$ are constant parameters characterizing gravitational potentials of specific BHs.

(ii) Accretion of matter onto the Black Hole model, according to models based on exponential increase as a function of time $t$,

$$M = M_0 + e^{k_1\left(\int_0^t r\, dt\right)^2}, \tag{102}$$

where $M_0 \in \mathbb{R}^+$ is the initial BH mass from which the accretion phenomenon is considered, and $k_1 \in \mathbb{R}^+$ is a parameter that defines the accretion rate. Thus, the drug administration $T \in \mathbb{R}^+$ can be established; (a) by defining $r = V - u$, such that $r \geq 0$ (Fig. 8d); (b) by introducing a sufficiently small $\varepsilon$ such that $\varepsilon \gtrsim 0$ ( $\epsilon \in \mathbb{R}$ ); (c) by establishing both $p$ and $q$ as even, i.e., $p \in \{2k : k \in \mathbb{N}^+\}, q \in \{2k : k \in \mathbb{N}^+\}$, to ensure that $\Phi$ has real solutions.

In an embodiment, a closed-loop feedback control system (Fig. 8d) incorporates a BH controller, which will provide a therapy $T$ based on current tumour $V$ values throughout the treatment.

The BH controller was then implemented using these two astrophysical phenomena,

$$T = \left(M_0 + e^{k_1\left(\int_0^t r\, dt\right)^2}\right)\frac{(k_2\, r)^{p-1}}{\left((k_3\, r)^q + r_0^q\right)^{\frac{p}{q}}}. \tag{103}$$

Tumour growth dynamics can be modelled as they have been following universal laws, and constitute a quite promising scientific support for improving cancer treatment. Included are the Gompertzian and the Logistic tumour growth dynamic laws: they are widely used models that have been demonstrating the ability to provide good fitting with clinical data, both for animal models and human patients. Based on the well-known model proposed by Hahnfeldt *et al.,* the following specific Gompertzian tumour growth model was used, which includes antiangiogenic dynamics and pharmacodynamics due to the administration of chemotherapeutic drugs (Model-1),

$$\dot{V} = -\xi\ln\left(\frac{V}{R}\right)V - \varphi VC \tag{104}$$

$$\dot{R} = bV - \left(\mu + dV^{\frac{2}{3}}\right)R - \eta RC$$

$$\dot{C} = -\lambda C + T,$$

where $V[\text{mm}^3]$ is the tumour volume, $R[\text{mm}^3]$ is the carrying capacity of the vasculature, $C[\text{mg/kg}]$ is the drug concentration, $T[\text{mg/kg}]$ is the drug administration, $\varphi[\text{conc}^{-1}\,\text{day}^{-1}]$ is cytotoxic effect on $V$, $\eta[\text{conc}^{-1}\,\text{day}^{-1}]$ cytotoxic effect on $R$, $\xi[\text{day}^{-1}]$ is tumour growth parameter, $b\,[\text{day}^{-1}]$ is angiogenic stimulation, $d\,[\text{mm}^{-2}\text{day}^{-1}]$ is the angiogenic inhibition, $\mu\,[\text{day}^{-1}]$ is the natural loss of endothelial support, and $\lambda\,[\text{day}^{-1}]$ is the drug elimination within the body.

This model was primarily parametrized according to a specific tumour dynamics widely used in this research field: $\xi = 0.192$, $b = 5.85$, $d = 0.00873$, $\mu = 0.02$, $\varphi = 4$, $\eta = 1$. $\lambda$ is a drug-specific parameter. These parameters were also primarily used in all models considered herein. A Gompertzian tumour growth model with generalized antiangiogenic dynamics was also tested (Model-2),

$$\dot{V} = -\xi \ln\left(\frac{V}{R}\right)V - \varphi VC \tag{105}$$

$$\dot{R} = bV^m - (\mu + dV^n)R - \eta RC$$

$$\dot{C} = -\lambda C + T,$$

where $1/3 \leq m \leq 4/3 (m \in \mathbb{R}^+)$ and $1/3 \leq n \leq 4/3 \ (n \in \mathbb{R}^+)$ are constant parameters defining a specific vasculature dynamics. Finally, the generalized Logistic tumour growth model (Model-3),

$$\dot{V} = \xi\left(1 - \left(\frac{V}{R}\right)^l\right)V - \varphi VC \tag{106}$$

$$\dot{R} = bV^m - (\mu + dV^n)R - \eta RC$$

$$\dot{C} = -\lambda C + T,$$

was also considered to analyze the performance of the BH controllers, where $1/5 \leq l \leq 5 (l \in \mathbb{R}^+)$ is a constant parameter defining a specific tumour growth dynamics.

The proposed solution of BH controllers are able to provide more robust dynamics that can be used in cancer treatments. It is proved herein that these controllers can reach equilibrium states $[V\ R\ C\ z]^T$.

It is also proved that the disclosed BH controllers can ensure stable equilibrium treatments, required to keep cancer volume V within the cancer event horizon, such that $V \to 0$ as $r \to 0$. The identification of the stability conditions is mandatory to find the necessary conditions for validating the dose administration as an effective treatment. Therefore, it was analyzed if the BH controllers, operating in a closed-loop feedback control scheme (Fig. 9) for the Gompertzian and Generalized Logistic tumour growth models, are able to change the tumour dynamics towards trapped regions where tumour volumes will not increase ($V_t \leq V_{t+1}$), and which conditions must be met for such purpose. Firstly, Model-1 (equation 106) was linearized, resulting in

$$\dot{V}_{Lt} = \Lambda_{t1} + \nabla_{VV-C}(V_{Lt} - V_t) + \nabla_{VR}(R_{Lt} - R_t) - \varphi(V_{Lt} - V_t)C_t \tag{107}$$

$$\dot{R}_{Lt} = \Lambda_{t2} + \nabla_{RV}(V_{Lt} - V_t) + \nabla_{RR-C}(R_{Lt} - R_t) - \eta(R_{Lt} - R_t)C_t$$

where $V_{Lt}$, $R_{Lt}$, $C_t$, $T_t$ are respectively the tumour volume, the vasculature capacity, the drug concentration and the drug administration of the linearized model at instant time $t$, $\Lambda_{t1} = -\xi \ln\left(\frac{V_t}{R_t}\right)V_t - \varphi V_t C_t$, $\Lambda_{t2} = bV_t - \left(\mu + dV_t^{\frac{2}{3}}\right)R_t - \eta R_t C_t$, $\nabla_{VV-C} = \xi\left(1 + \ln\left(\frac{V_t}{R_t}\right)\right)$, $\nabla_{RR-C} = \mu + dV_t^{\frac{2}{3}}$, $\nabla_{VR} = \frac{\xi V_t}{R_t}$, $\nabla_{RV} = b - \frac{2}{3}dR_t V_t^{-\frac{1}{3}}$. Taking $z = \int_0^t r\ dt = \int_0^t (V_{Lt} - u + \epsilon)\ dt$, the BH1 controller is integrated into a closed-loop system $f_t$ defined as

$$\dot{\mathbf{x}}_t = \boldsymbol{f}_t(t, \mathbf{x}_t) = \begin{bmatrix} f_{t1}(t, \mathbf{x}_t) \\ f_{t2}(t, \mathbf{x}_t) \\ f_{t3}(t, \mathbf{x}_t) \\ f_{t4}(t, \mathbf{x}_t) \end{bmatrix} \tag{108}$$

$$= \begin{cases} \dot{V}_{Lt} = \Lambda_{t1} + \nabla_{VV-C}(V_{Lt} - V_t) + \nabla_{VR}(R_{Lt} - R_t) - \varphi(V_{Lt} - V_t)C_{BH1-t}, \\ \dot{R}_{Lt} = \Lambda_{t2} + \nabla_{RV}(V_{Lt} - V_t) + \nabla_{RR-C}(R_{Lt} - R_t) - \eta(R_{Lt} - R_t)C_{BH1-t}, \\ \dot{C}_{BH1-t} = -\lambda C_{BH1-t} + \dfrac{\left(M_0 + e^{k_1 z^2}\right)\left(k_2(V_{Lt} - u)\right)^{p-1}}{\left(\left(k_3(V_{Lt} - u)\right)^q + r_0^q\right)^{\frac{p}{q}}}, \\ \dot{z} = V_{Lt} - u + \epsilon. \end{cases}$$

**Theorem 1**. The BH controller provides a unique treatment for the initial condition $\mathbf{x}_t(t_0) = [V_{Lt_0}\ R_{Lt_0}\ C_{BH1-t_0}\ z_0]^T$, $\forall t \in [t_0, t_1]$, and

$$\mathbf{x}_t^* = [V_{Lt}^*\quad R_{Lt}^*\quad C_{BH1-t}^*\quad z^*]^T = \tag{109}$$

$$\left[u + \epsilon \quad -\frac{r_{10}}{2} \pm \sqrt{r_{10}^2 - 4r_{11}} \quad \frac{r_{12}}{\varphi V_t + \eta\left(\frac{r_{10}}{2} \pm \sqrt{r_{10}^2 - 4r_{11}} + R_t\right)} \quad \pm\sqrt{\frac{1}{k_1}\ln\left(\frac{r_{13}}{r_{14}} - M_0\right)}\right]^T$$

is an equilibrium state of the closed system (10), where

$$r_{10} = \frac{\varphi V_t \nabla_{RR-C} + \eta\Lambda_{t1} - \eta V_t \nabla_{VV-C} - 2\eta R_t \nabla_{VR}}{\eta \nabla_{VR}}, \tag{110}$$

$$r_{11} = \frac{\varphi V_t \Lambda_{t2} - \varphi V_t^2 \nabla_{RV} - \varphi V_t R_t \nabla_{RR-C} - \eta R_t \Lambda_{t1} + \eta V_t R_t \nabla_{VV-C} + \eta \nabla_{VR} R_t^2}{\eta \nabla_{VR}},$$

$$r_{12} = V_t(\nabla_{RV} + \nabla_{VV-c}) + \left(\frac{r_{10}}{2} \pm \sqrt{r_{10}^2 - 4r_{11}} + R_t\right)(\nabla_{VR} + \nabla_{RR-c}) - (\Lambda_{t1} + \Lambda_{t2}),$$

$$r_{13} = \lambda\left((k_3\epsilon)^q + r_0^q\right)^{\frac{p}{q}}\left(V_t(\nabla_{RV} + \nabla_{VV-c}) + \left(\frac{r_{10}}{2} \pm \sqrt{r_{10}^2 - 4r_{11}} + R_t\right)(\nabla_{VR} + \nabla_{RR-c})\right.$$

$$\left. - (\Lambda_{t1} + \Lambda_{t2})\right),$$

$$r_{14} = (k_2\epsilon)^{p-1}\left(\varphi V_t + \eta\left(\frac{r_{10}}{2} \pm \sqrt{r_{10}^2 - 4r_{11}} + R_t\right)\right),$$

and ensuring $r_{10}^2 - 4r_{11} > 0$, $\varphi V_t + \eta\left(\frac{r_{10}}{2} \pm \sqrt{r_{10}^2 - 4r_{11}} + R_t\right) \neq 0$ and $\frac{1}{k_1}\ln\left(\frac{r_{12}}{r_{13}} - M_0\right) > 0$.

**Theorem 2.** The stability of the equilibrium point $\mathbf{x}_t^*$ is ensured if four conditions are met:

Condition 1:

$$a_1 = \frac{r_{12}(\varphi + \eta)}{c_1} - \nabla_{VV-C} - \nabla_{RR-C} + \lambda^2 > 0 \tag{111}$$

where

$$c_1 = \varphi V_t + \eta\left(\frac{r_{10}}{2} \pm \sqrt{r_{10}^2 - 4r_{11}} + R_t\right).$$

Condition 2:

$$a_3 = \lambda c_6 \pm \frac{2k_1\varphi M_0 c_5 c_7 k_2{}^{p-1}}{c_4} \tag{112}$$

$$+\frac{\lambda r_{12}}{c_1\epsilon}\left(-\nabla_{VR}\eta c_2 c_{10} + \varphi\nabla_{RR-C}c_{11} - c_8\epsilon + \frac{\varphi\eta r_{12}\epsilon}{c_1} \pm 2k_1\varphi c_5(\epsilon - V_t)\epsilon + \eta\varphi c_3 c_7 c_9 r_{12}k_3{}^q\right)$$

$$> 0$$

where

$$c_2 = \frac{c_1 - \varphi V_t}{\eta}, \; c_3 = \left((k_3\epsilon)^q + r_0^q\right)^{-1}, \; c_4 = \left((k_3\epsilon)^q + r_0^q\right)^{\frac{p}{q}}, \; c_5 = \sqrt{\frac{1}{k_1}\ln\left(\frac{r_{13}}{r_{14}} - M_0\right)}, \; c_6 =$$

$$\nabla_{VV-C}\nabla_{RR-C} - \nabla_{VR}\nabla_{RV}, \; c_7 = (\epsilon)^{q-1}(\epsilon - V_t), \; c_8 = \eta\nabla_{VV-C} + \varphi\nabla_{RR-C}, \; c_9 = \frac{1}{\epsilon} - \frac{p}{c_1}, \; c_{10} = p -$$

$$1 - pc_3 k_3{}^q \epsilon^q, \; c_{11} = (p-1)(\epsilon - V_t) - \frac{c_3 c_7 p k_3{}^q}{\epsilon}.$$

Condition 3:

$$a_4 = \frac{\pm 2k_1\lambda r_{12}c_2 c_5 c_{13}}{c_1} \pm \frac{2k_1 M c_5 c_{14}k_2{}^{p-1}}{c_4} > 0 \tag{113}$$

where

$$c_{12} = \nabla_{RR-C} + \eta r_{12}, c_{13} = \varphi(\epsilon - V_t) + \eta\nabla_{VR}, c_{14} = \varphi c_7 c_{12} + \eta\nabla_{VR}c_2\epsilon^{p-1}.$$

Condition 4

$$a_1 a_2 a_3 - a_1^2 a_4 - \lambda a_3^2 > 0 \tag{114}$$

where

$$a_2 = c_6 - \lambda(\nabla_{VV-C} + \nabla_{RR-C}) + \frac{r_{12}c_{15}}{c_1} \; and \; c_{15} = \lambda\varphi + \lambda\eta - c_8 + \frac{\varphi\eta r_{12}}{c_1} - \frac{\lambda\varphi c_{10}(\epsilon - V_t)}{\epsilon}.$$

Concerning the other models, their linearization conducts to few changes, namely:

Model-2 (equation 105): $\Lambda_{t2} = bV_t^m - (\mu + dV_t^n)R_t - \eta R_t C_t$ ; $\nabla_{RV} = bmV_t^{m-1} - ndR_t V_t^{n-1}$ ; and

$\nabla_{RR\text{-}C} = \mu + dV_t^n$.

Model-3 (equation 107):
$$\Lambda_{t1} = \xi \left(1 - \left(\frac{V_t}{R_t}\right)^l\right) V_t - \varphi V_t C_t \quad ; \quad \Lambda_{t2} = bV_t^m - (\mu + dV_t^n)R_t - \eta R_t C_t \; ;$$

$$\nabla_{VR} = -\xi l \left(\frac{V_t}{R_t}\right)^{l+1} \; ;$$

$$\nabla_{VV\text{-}C} = \xi \left(1 - \left(\frac{V_t}{R_t}\right)^l (1+l)\right) \; ; \; \nabla_{RV} = bmV_t^{m-1} - ndR_t V_t^{n-1} \; ; \; \nabla_{RR-C} = \mu + dV_t^n$$

These results allow clinicians to carry out the patient screening process, as well as to select the most effective BH controller, as described in **Protocol 1.**

| **Protocol 1** \| Patient screening process. |
|---|
| 1: *a Identify initial tumour volume $V_0$ and $R_0$:* |
| 2: Measure $V_0$ and $R_0$ using clinical imaging or *in situ* sensing |
| 3. Define treatment trajectory for tumour dynamics |
| 4. **for** each tumour growth model-i **do** |
| 5. *Find parameters $\xi$, $b$, $d$ , $\mu$ , $\varphi$ , $\eta$* |
| 6. *Calculate fitting score to clinical data* |
| 7. **end** |
| 8. Select the tumour growth model and related parameters with the highest fitting score |
| 9. *# Calculate the equilibrium states:* |
| 10. Define maximum dose administration $T_{max}$, maximum drug concentration $C_{max}$, and treatment time $t_{max}$ |
| 11. Optimize BH control parameters $r_0$, $M_0$, $k_1$, $k_2$, $k_3$ |
| 12 Calculate $\mathbf{x}_t^*$ for the BH controller when $t = t_{max}$ |
| 13. Calculate $T_{BH-t_{max}}^*$ |
| 14. if $C_{BH-t_{max}}^* > C_{max}$ and $T_{BH-t_{max}}^* > T_{max}$ **then** |
| 15. *Exclude treatment using the BH controller* |
| 16. **end** |
| 17. *# Test the stability conditions for the BH controller if it was not excluded* |
| 18. **if** $a_1 > 0$ and $a_3 > 0$ and $a_4 > 0$ and $a_1 a_2 a_3 - a_1^2 a_4 - \lambda a_3^2 > 0$ **then** |
| 19. *Compute the average dose administration $T_{avg}$ from $t = t_0$ to $t = t_{max}$* |
| 20. *Compute the average drug concentration $C_{avg}$ from $t = t_0$ to $t = t_{max}$* |
| 21. *Compute the treatment duration* |
| 22. **else** |
| 23. *Exclude treatment using the BH controller* |
| 24. end |

The BH controller was able to significantly provide a complete response (target tumour volumes < 10 $mm^3$) for multiple tumour-treatment scenarios, namely for:

(i) Two tumours with very different dimensional characterizations: a small volumetric tumour ($V_0 = 200$ $mm^3$; $R_0 = 625$ $mm^3$) and a much larger tumour ($V_0 = 12732$ $mm^3$; $R_0 = 15915$ $mm^3$).
(ii) Uncertain tumour dynamics, expressed by multivalued model parameters (Fig. 10) for: (a) for multiple combinations of parameters, namely the binomial combinations $(\xi \mid \mu)$, $(d \mid b)$, $(\varphi \mid \eta)$, $(\xi \mid \eta)$, $(\varphi \mid d)$, $(n \mid m)$, $(l \mid m)$ and $(l \mid m)$; (b) for $1 \times 10^5$ random combination of parameters $(\xi, b, d, \mu, \varphi, \eta, n, m, l)$. According to clinical data, each model parameter can exhibit a significant range of variation that can extend from 0.2-fold to 5-fold the primarily considered parametrization. Therefore, a variable factor in the range $0.2 \le factor \le 5$ was used to establish $factor \times parameter$ (e.g. the $\xi$ parameter was varied according to $factor \times \xi$, in which $0.2 \le factor \le 5$). Only one exception was accounted: the factor of $\varphi$ was taken in the range $1 \le factor \le 5$, to assume cytotoxic effects $\varphi > 1$, as established before.

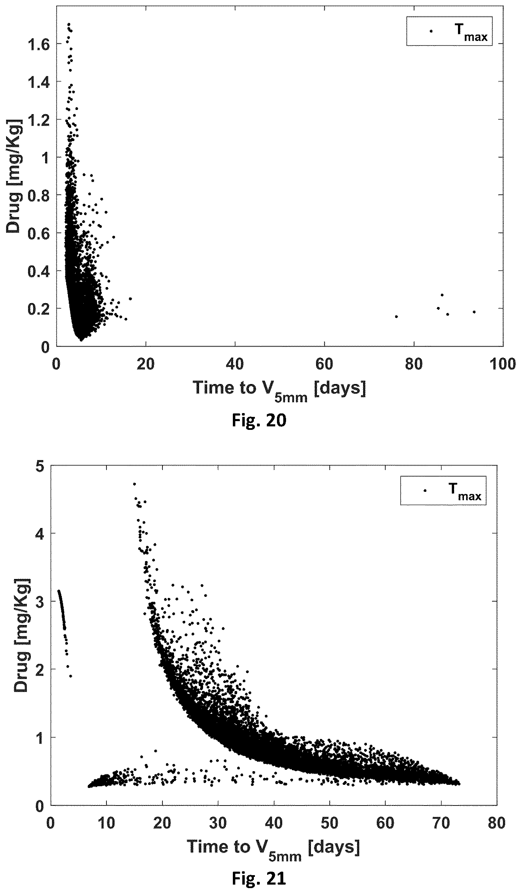

(iv) Two chemotherapy drugs (see Figures 20 and 21) with very different drug elimination rate $\lambda$: Cyclophosphamide ($\lambda$ = 4.16) and Atezolizumab ($\lambda$ = 0.0256). Concerning Cyclophosphamide, short time dose administration can exceed 100 mg/kg, although intermediate and low dose is usually considered up to 25 mg/kg and up to 3 mg/kg, respectively. Concerning Atezolizumab, the usual recommendation is within the range between 1 mg/kg and 21 mg/kg.

Optimization of BH1 and BH2 controllers were carried out only for the primarily parametrization of tumour models, but ensuring: (a) low dose administration (Cyclophosphamide: $T \le 3\ mg/Kg$; Atezolizumab: $T \le 1\ mg/kg$) for these primary parameters; (b) maximum dose administration (Cyclophosphamide: $T \le 25\ mg/Kg$; Atezolizumab: $T \le 21\ mg/kg$ in 21 days) is not exceeded for multivalued scenarios modeling uncertainty.

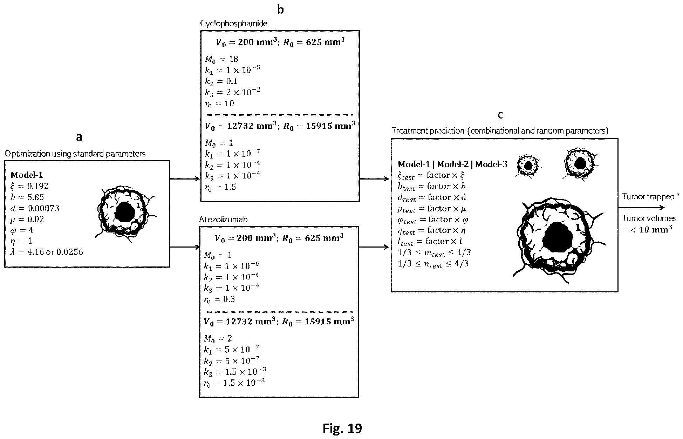

**Figure 19** shows an embodiment of optimization and response for multiple tumour dynamics: a, optimization using standard parameters; b, optimized BH controller parameters for different tumour and vasculature volumes, as well as for different chemotherapy drugs; c, target tumour volumes < 10 $mm^3$ both for combinational and random parameters.

This description does not restrict the realization presented in this document. Anyone with average knowledge in this field can foresee many possibilities to modify it. The following claims additionally define preferred realizations.

The term "comprising" whenever used in this document is intended to indicate the presence of stated features, integers, steps, components, but not to preclude the presence or addition of one or more other features, integers, steps, components, or groups thereof.

The disclosure should not be seen in any way restricted to the embodiments described and a person with ordinary skill in the art will foresee many possibilities to modifications thereof. The above-described embodiments are combinable.

The following claims further set out particular embodiments of the disclosure.

References

1. Behranvand N., et al. Chemotherapy: a double-edged sword in cancer treatment. Cancer Immunol. Immunother. 71, 507-526 (2022).

2. Sung, H., et al. Global Cancer Statistics 2020: GLOBOCAN Estimates of Incidence and Mortality Worldwide for 36 Cancers in 185 Countries, CA Cancer J. Clin. 71, 209-249 (2021).

3. Ferlay, J., et al. Cancer statistics for the year 2020: An overview, Int. J. Cancer 149, 778-789 (2021).

4. Dyba, T. et al. The European cancer burden in 2020: Incidence and mortality estimates for 40 countries and 25 major cancers, Eur. J. Cancer 157, 308-347 (2021).

5. Beeghly, G., Shimpi, A., Riter, R., Fischbach, C. Measuring and modelling tumour heterogeneity across scales, Nat. Rev. Bioeng. 1, 712-730 (2023).

6. Mizrahi, J., Surana, R., Valle, J., Shroff,R. Pancreatic cancer. Lancet 395, 2008-2020 (2020).

**Claims**

**1.** Control method for trajectory tracking of a dynamic system (2) having an input $u(t)$ and having an output $y(t)$, comprising the steps:

obtaining a signal error $e(t)$ from the difference between a reference signal $u(t)$ and the output signal $y(t)$;

obtaining, by a controller (1,4), a control value $c_{BN}(t)$ determined by a black hole (BH) gravitational potential field of a black hole mass $M(t)$, wherein the reference signal $u(t)$ is the black hole centre, wherein the signal error $e(t)$ is a distance a current position $y(t)$ to the black hole centre $u(t)$,

wherein the output signal $y(t)$ is a current position within a black hole Schwarzschild radius of the black hole, and wherein the black hole mass $M(t)$ includes a factor of matter accretion; and

inputting the control value $c_{BN}(t)$ into the dynamic system (2).

2. Control method according to the previous claim, comprising the steps:

   obtaining the signal error $e(t)$ from the difference between the reference signal $u(t)$ and the output signal $y(t)$;
   obtaining, by the controller (1,4), the control value $c_{BN}(t)$ determined by the equation:

   $$c_{BN}(t) = M(t)\frac{\left(ae(t)\right)^{p-1}}{\left(\left(ae(t)\right)^{q} + r_0^{q}\right)^{p/q}}$$

   wherein $M(t)$ corresponds to the factor of matter accretion, $p$, $q$ and $a$ are pre-defined parameters to define a convergence potential, and $r_0$ is a pre-defined constant to ensure non-singular solution or solutions, and wherein $p$ and $q$ are even and positive; and
   inputting the control value $c_{BN}(t)$ into the dynamic system (2).

3. Control method according to any of the previous claims wherein the factor of matter accretion $M(t)$ is determined by $M(t) = M_0 + k_1 exp(k_2(\int e\, dt)^2)$, wherein $M_0$ is an initial mass parameter of the BH controller, and $k_1$ and $k_2$ are predefined convergence parameters.

4. Control method according to any of the previous claims comprising a hyperparameter tuning step, in particular a preparatory hyperparameter tuning step, in particular using a Nelder-Mead simplex and/or BH optimisation algorithm.

5. Control method according to any of the previous claims wherein the dynamic system comprises a tumour growth dynamic model, wherein the input $u(t)$ is a tumour treatment parameter and the output $y(t)$ is a tumour growth metric, in particular a tumour volume.

6. Controller (1,4) for trajectory tracking of a dynamic system (2) having an input $u(t)$ and having an output $y(t)$, comprising a data processor arranged to carry out the steps of:

   obtaining a signal error $e(t)$ from the difference between a reference signal $u(t)$ and the output signal $y(t)$;
   obtaining, by a controller (1,4), a control value $c_{BN}(t)$ determined by a black-hole gravitational potential field of a black hole mass $M(t)$, wherein the reference signal $u(t)$ is the black hole centre, wherein the signal error $e(t)$ is a distance to the black hole centre $u(t)$, wherein the output signal $y(t)$ is a current position within a black hole Schwarzschild radius of the black hole, and wherein the black hole mass $M(t)$ includes a factor of matter accretion $M(t)$ and
   inputting the control value $c_{BN}(t)$ into the dynamic system (2).

7. Controller according to the previous claim wherein the data processor is further arranged to carry out the steps of:

   obtaining the signal error $e(t)$ from the difference between the reference signal $u(t)$ and the output signal $y(t)$;
   obtaining, by the controller (1,4), the control value $c_{BN}(t)$ determined by the equation:

   $$c_{BN}(t) = M(t)\frac{\left(ae(t)\right)^{p-1}}{\left(\left(ae(t)\right)^{q} + r_0^{q}\right)^{p/q}}$$

   wherein $M(t)$ corresponds to the factor of matter accretion, $p$, $q$ and $a$ are pre-defined parameters to define a convergence potential, and $r_0$ is a pre-defined constant to ensure non-singular solution or solutions, and wherein $p$ and $q$ are even; and
   inputting the control value $c_{BN}(t)$ into the dynamic system (2).

8. Controller according to any of the claims 5-6 wherein the factor of matter accretion $M(t)$ is determined by $M(t) = M_0 + k_1 exp(k_2(\int e\, dt)^2)$, wherein $M_0$ is an initial mass parameter of the BH controller, and $k_1$ and $k_2$ are predefined convergence parameters.

9. Dynamic system (2) comprising a controller for trajectory tracking of the dynamic system (2) of any of the claims 5-7.

10. Dynamic system (2) according to the previous claim wherein the dynamic system is a linear system or a non-linear system linearized at particular operating points.

11. Dynamic system (2) according to the claim 8 wherein the dynamic system is a non-linear system.

12. Dynamic system according to any of the claims 8-11 further comprising a tumour growth dynamic model, wherein the input u(t) is a tumour treatment parameter and the output y(t) is a tumour growth metric, in particular a tumour volume.

**Fig. 1**

**Fig. 2**

**Fig. 3**

**Fig. 4**

**Fig. 5**

**Fig. 6**

**Fig. 7**

**Fig. 8**

**Fig. 9**

**Fig. 10**

(a)

(b)

(c)

(d)

(e)

(f)

**Fig. 11**

(a)

(b)

**Fig. 12**

Time (s)

**Fig. 13**

Fig. 14

Fig. 15

Fig. 16

**Fig. 17**

**Fig. 18**

**b**

Cyclophosphamide

$V_0 = 200 \text{ mm}^3$; $R_0 = 625 \text{ mm}^3$

$M_0 = 18$
$k_1 = 1 \times 10^{-5}$
$k_2 = 0.1$
$k_3 = 2 \times 10^{-2}$
$r_0 = 10$
- - - - - - - - - - - - - - - - - -
$V_0 = 12732 \text{ mm}^3$; $R_0 = 15915 \text{ mm}^3$

$M_0 = 1$
$k_1 = 1 \times 10^{-7}$
$k_2 = 1 \times 10^{-4}$
$k_3 = 1 \times 10^{-4}$
$r_0 = 1.5$

**c**

Treatment prediction (combinational and random parameters)

**Model-1 | Model-2 | Model-3**
$\xi_{test} = \text{factor} \times \xi$
$b_{test} = \text{factor} \times b$
$d_{test} = \text{factor} \times d$
$\mu_{test} = \text{factor} \times \mu$
$\varphi_{test} = \text{factor} \times \varphi$
$\eta_{test} = \text{factor} \times \eta$
$l_{test} = \text{factor} \times l$
$1/3 \leq m_{test} \leq 4/3$
$1/3 \leq n_{test} \leq 4/3$

Tumor trapped *

Tumor volumes
< 10 mm³

**a**

Optimization using standard parameters

**Model-1**
$\xi = 0.192$
$b = 5.85$
$d = 0.00873$
$\mu = 0.02$
$\varphi = 4$
$\eta = 1$
$\lambda = 4.16 \text{ or } 0.0256$

Atezolizumab

$V_0 = 200 \text{ mm}^3$; $R_0 = 625 \text{ mm}^3$

$M_0 = 1$
$k_1 = 1 \times 10^{-6}$
$k_2 = 1 \times 10^{-4}$
$k_3 = 1 \times 10^{-4}$
$r_0 = 0.3$
- - - - - - - - - - - - - - - - - -
$V_0 = 12732 \text{ mm}^3$; $R_0 = 15915 \text{ mm}^3$

$M_0 = 2$
$k_1 = 5 \times 10^{-7}$
$k_2 = 5 \times 10^{-7}$
$k_3 = 1.5 \times 10^{-3}$
$r_0 = 1.5 \times 10^{-3}$

**Fig. 19**

Fig. 20

Fig. 21

Europäisches
Patentamt

European
Patent Office

Office européen
des brevets

# EUROPEAN SEARCH REPORT

**Application Number**

EP 24 15 0601

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | YUAN CAO: "PARAMETER-VARYING ARTIFICIAL POTENTIAL FIELD CONTROL OF VIRTUAL COUPLING SYSTEM WITH NONLINEAR DYNAMICS", FRACTALS., vol. 30, no. 02, 1 March 2022 (2022-03-01), XP093162443, SG ISSN: 0218-348X, DOI: 10.1142/S0218348X22400990 Retrieved from the Internet: URL:https://www.worldscientific.com/doi/pdf/10.1142/S0218348X22400990> * 3- 5 * | 1-12 | INV. G05B13/04 G05B17/00 ADD. G06F17/11 |

TECHNICAL FIELDS
SEARCHED      (IPC)

G05B
G06F
G16H

The present search report has been drawn up for all claims

| Place of search | Date of completion of the search | Examiner |
|---|---|---|
| The Hague | 14 May 2024 | De Porcellinis, S |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
 
& : member of the same patent family, corresponding
    document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 110880031 A

**Non-patent literature cited in the description**

- **BEHRANVAND N. et al.** Chemotherapy: a double-edged sword in cancer treatment. *Cancer Immunol. Immunother.,* 2022, vol. 71, 507-526
- **SUNG, H. et al.** Global Cancer Statistics 2020: GLOBOCAN Estimates of Incidence and Mortality Worldwide for 36 Cancers in 185 Countries. *CA Cancer J. Clin.,* 2021, vol. 71, 209-249
- **FERLAY, J. et al.** Cancer statistics for the year 2020: An overview. *Int. J. Cancer,* 2021, vol. 149, 778-789
- **DYBA, T. et al.** The European cancer burden in 2020: Incidence and mortality estimates for 40 countries and 25 major cancers. *Eur. J. Cancer,* 2021, vol. 157, 308-347
- **BEEGHLY, G. ; SHIMPI, A. ; RITER, R. ; FISCHBACH, C.** Measuring and modelling tumour heterogeneity across scales. *Nat. Rev. Bioeng.,* 2023, vol. 1, 712-730
- **MIZRAHI, J. ; SURANA, R. ; VALLE, J. ; SHROFF,R.** Pancreatic cancer. *Lancet,* 2020, vol. 395, 2008-2020